# EUROPEAN PATENT APPLICATION

(11) **EP 3 666 757 A1**
(43) Date of publication of application: **17.06.2020**
(21) Application number: 18211702.8
(22) Date of filing: 11.12.2018
(51) Int. Cl.: C07D 211/14, C07D 211/58, C07D 401/06, C07B 41/06

(54) **PROCESS FOR PREPARING A PIPERIDIN-4-ONE**

(71) Applicant: Karl-Franzens-Universität Graz, 8010 Graz (AT)
(72) Inventor: Seebacher, Werner, 8044 Graz-Mariatrost (AT)
(74) Representative: Pföstl, Andreas

(57) **Abstract**

The present invention relates to a process for preparing a pi-peridin-4-one comprising a step of reducing a tetrahydropyridin-4-ylidene ammonium salt of formula (I) 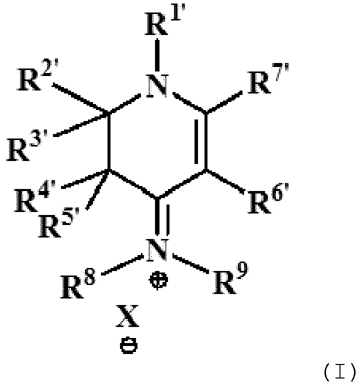 to a compound of formula (II) 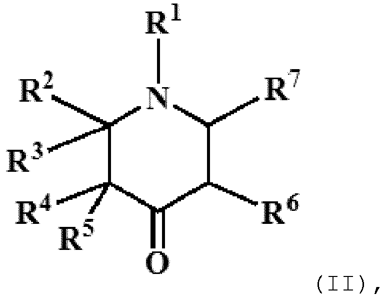 as well as a compound according to formula (II) and a method of derivatizing a piperidin-4-one.

## Description

### TECHNICAL FIELD

The present invention relates to a process for preparing a piperidin-4-one and novel piperidin-4-ones as well as a method of derivatizing a piperidin-4-one.

### BACKGROUND ART

Piperidin-4-ones can act as useful intermediates for the syntheses of 4-aminopiperidine therapeutic compounds (Sun & Scott, (2011) 2:638-643). Examples of this compound class include important drugs such as fentanyl, alfentanil, sufentanil, remifentanil, bamipine, thenaldine, astemizol, indoramin, diphenylpyraline,pimozide, domperidone, loperamide etc. also the 4-aryl,4-hydroxypiperidine drug haloperidol is obtainable from the piperidin-4-one intermediate.

As valuable building blocks piperidin-4-ones can be used for the preparation of
a) nitrogen heterocycles (see e.g. M. Srinivasan et al., 2007, Tetrahedron, 63: 2865-2874; R.S. Alekseyev et al., 2011, Chemistry of Heterocyclic Compounds 47: 584-596; T. Troxler, 2013, Bioorganic & Medicinal Chemistry Letters 23: 4085-4090; Z. Wang et al., 2017, European Journal of Medicinal Chemistry 139: 128-152)
b) bicyclic systems like bispidines (N. Shi et al., 2011, Youji Huaxue 31: 497-504)
c) spiro compounds (H. Prevet et al., 2016, Tetrahedron Letters 57: 2888-2894; US 2014/0113895; A. Mallinger, 2016, Journal of Medicinal Chemistry 59:1078-1101)
d) metal complexes (A. Cachapa et al., 2006, Polyhedron 25: 3366-3378)
d) antitumor agents (WO 2016/191871)
e) alkaloids (M.E. Kuehne et al., 1987, Journal of Organic Chemistry 52: 347-353).

Due to the pharmacological potential or biological activity of piperidin-4-ones and the products, which are prepared from piperidin-4-ones, it is of high interest to have a large variety of potential substitution patterns. Especially, piperidin-4-ones with substitution at the carbon atom adjacent to the nitrogen are rarely available. Thus, 2-substituted derivatives of most of the drugs or active substances, which can be made from piperidin-4-ones have not been investigated until now. To cover a complete structure-activity relationship of a substance class it is essential to investigate all relevant sites of substitution.

Usually, piperidones are prepared by a one-step approach involving a condensation of C-H acidic compounds with aldehydes and amines (P. Petrenko-Kritschenko, M. Lewin, 1907, Ber Chem 40: 2882-2885). This approach allows to obtain 2,6-disubstituted-piperidone compounds, as for example shown in P. Perumal et al., 2013, Int J Pharm Pharm Sci 5: Suppl 2, 317-321. However, especially asymmetrical substitution at the position adjacent to the nitrogen is difficult with known approaches.

Individually, 2,2-dimethylpiperidin-4-ones have been described (R. S. Vartanyan et al., 1984, Arm Khim Zhur 37: 724-725.; R. E. Markaryan, et al., 1988, Arm Khim Zhur 41:311-313) .

However, 2-substituted piperidin-4-ones have been only investigated to a very limited extent. This is for example reflected by the fact that the CAS SciFinder Database lists about 2555 reactions for N-benzyl-piperidin-4-one, whereas for the related N-benzyl-2,2-dimethyl-piperidin-4-one only 28 reactions are reported.

It is therefore an object of the present invention to provide alternative processes for preparing piperidin-4-ones or derivatives thereof, and especially to provide processes, wherein piperidin-4-ones with various substitution patterns can be obtained, preferably 2-substituted piperidin-4-ones.

### SUMMARY OF THE INVENTION

The present invention relates to a process for preparing a piperidin-4-one comprising a step of reducing a tetrahydropyridin-4-ylidene ammonium salt of formula (I) 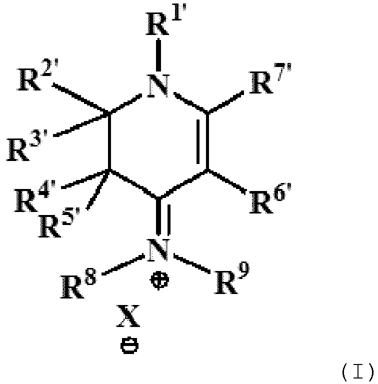 to a compound of formula (II) 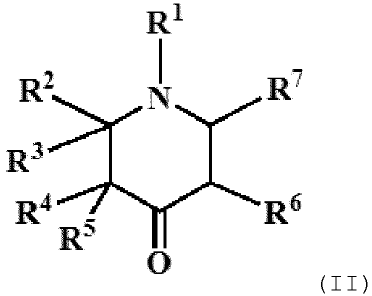 wherein R¹, R², R³, R⁴, R⁵, R⁶, and R⁷ are independently from each other substituents selected from the group consisting of
a) hydrogen,
b) C₁-C₁₀-alkyl,
c) C₆-C₁₄-aryl,
d) heteroaryl, wherein the heteroaryl is a 5 to 14 membered aromatic ring system with at least one heteroatom selected from the group consisting of N, O, and S atoms,
e) C₅-C₁₄-aryl-C₁-C₄-alkyl,
f) heteroaryl-C₁-C₄-alkyl,
g) C₂-C₁₀-alkenyl containing one or more double bonds,
h) C₂-C₁₀-alkinyl containing one or more triple bonds,
i) C₅-C₁₄-aryl-C₂-C₄-alkenyl containing one or more double bonds,
j) C₅-C₁₄-aryl-C₂-C₄-alkinyl containing one or more triple bonds,
k) heteroaryl-C₂-C₄-alkenyl containing one or more double bonds,
l) heteroaryl-C₂-C₄-alkinyl containing one or more triple bonds, wherein the heteroaryl is 5 to 14 membered aromatic ring system with at least one heteroatom selected from the group consisting of N, O, and S atoms,
and wherein optionally one or more of substituents b) to 1) are substituted,
R⁸ and R⁹ are independently from each other substituents selected from the group consisting of a) to 1) as defined above, or
R⁸ and R⁹ form together with the nitrogen to which they are bound a 3 to 7 membered unsubstituted or substituted non-aromatic heterocyclic ring, wherein the non-aromatic heterocyclic ring optionally contains one or more additional heteroatoms selected from the group consisting of N, O, and S atoms,
wherein R^{1'}, R^{2'}, R^{3'}, R^{4'}, R^{5'}, R^{6'}, and R^{7'} independently of each other are identical to R¹, R², R³, R⁴, R⁵, R⁶, and R⁷, respectively, or R^{1'}, R^{2'}, R^{3'}, R^{4'}, R^{5'}, R^{6'}, and R^{7'} are precursors of R¹, R², R³, R⁴, R⁵, R⁶, and R⁷, respectively, which are reducible to R¹, R², R³, R⁴, R⁵, R⁶, and R⁷, respectively,
and X⁻ is an anion.

It was surprisingly found that the reduction of the tetrahydropyridin-4-ylidene ammonium salts of the general formula (I) results in piperidin-4-ones instead of the corresponding piperidine-ring.

Another aspect of the present invention relates to a piperidin-4-one compound of formula (II) 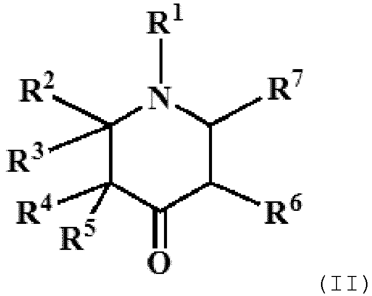 wherein R¹, R², R³, R⁴, R⁵, R⁶, and R⁷ are independently from each other substituents selected from the group consisting of
a) hydrogen,
b) C₁-C₁₀-alkyl,
c) C₆-C₁₄-aryl,
d) heteroaryl, wherein the heteroaryl is 5 to 14 membered aromatic ring system with at least one heteroatom selected from the group consisting of N, O, and S atoms,
e) C₅-C₁₄-aryl-C₁-C₄-alkyl,
f) heteroaryl-C₁-C₄-alkyl,
g) C₂-C₁₀-alkenyl containing one or more double bonds,
h) C₂-C₁₀-alkinyl containing one or more triple bonds,
i) C₅-C₁₄-aryl-C₂-C₄-alkenyl containing one or more double bonds,
j) C₅-C₁₄-aryl-C₂-C₄-alkinyl containing one or more triple bonds,
k) heteroaryl-C₂-C₄-alkenyl containing one or more double bonds,
l) heteroaryl-C₂-C₄-alkinyl containing one or more triple bonds, wherein the heteroaryl is 5 to 14 membered aromatic ring system with at least one heteroatom selected from the group consisting of N, O, and S atoms,
and wherein optionally one or more of substituents b) to l) are substituted,
and wherein at least one of R², R³, or R⁷ is not hydrogen, preferably at least one of R² and R³ is not hydrogen.

The piperidin-4-one compound of formula (II) according to the invention, wherein at least one of R², R³, or R⁷ is not hydrogen, preferably at least one of R² and R³ is not hydrogen, is a 2-substituted piperidin-4-one. Such a compound can be obtained with the process according to the present invention. A compound of formula (II) according to the present invention, wherein at least one of R², R³, or R⁷ is not hydrogen, is referred to herein as a compound according to the invention.

Another aspect of the invention relates to a method of derivatizing a piperidin-4-one comprising the step of subjecting a compound of formula (II) obtainable by a process according to the present invention as described above or a compound according to the present invention to a reaction selected from the group of
- reductive amination to obtain a compound according to formula (XIX) 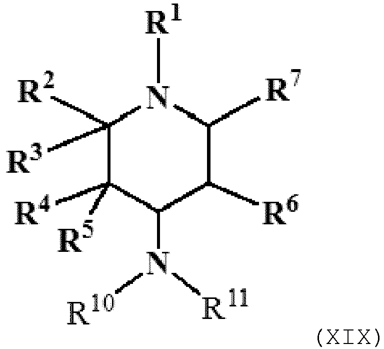
- reduction and etherification to obtain a compound according to formula (XX) 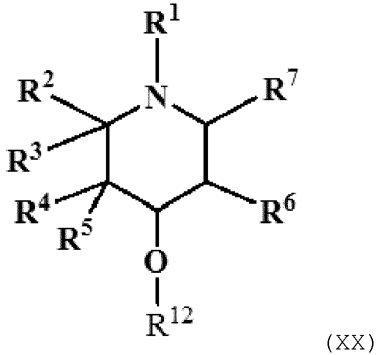
- reacting by Grignard reaction to obtain a compound according to formula (XXI) 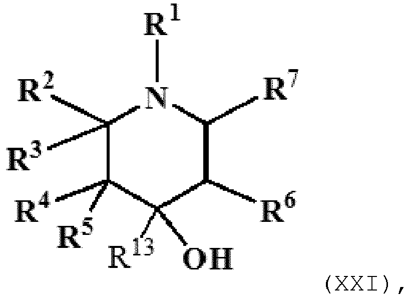
wherein R¹⁰, R¹¹, R¹² and R¹³ are independently of each other hydrogen or an organic moiety.

### SHORT DESCRIPTION OF THE FIGURES

Fig. 1 shows a reaction scheme of a process of the present invention.
Fig. 2 shows a reaction scheme of a process of the present invention.

### DESCRIPTION OF EMBODIMENTS

It was surprisingly found that piperidin-4-ones can be obtained by reducing tetrahydropyridin-4-ylidene ammonium salts of the general formula (I), e.g. by the reaction according to the following scheme:

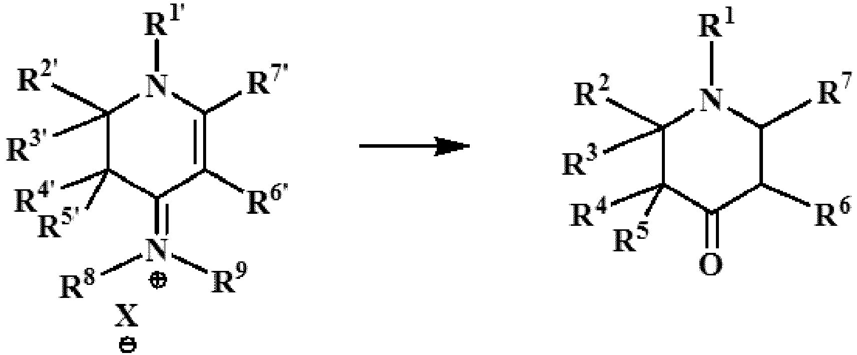

According to a particularly preferred embodiment of the present invention the reducing step in the process of the present invention is performed using a hydrogenation agent. It turned out that the use of hydrogenation agents results in a decreased formation of undesired by-products such as corresponding amines or diamines or 4-hydroxypiperidine, which could be expected in the course of the reduction of tetrahydropyridin-4-ylidene ammonium salts.

According to a preferred embodiment of the present invention, the hydrogenation agent is an aluminium hydride, preferably selected from the group consisting of sodium bis(2-methoxyethoxy)aluminium hydride (known as Red-Al®) and lithium aluminium hydride, more preferably lithium aluminium hydride.

In a preferred embodiment of the present invention, the molar ratio between the hydrogenating agent and the amount of the tetrahydropyridin-4-ylidene ammonium salt of formula (I) is in the range of from 2:1 to 6:1, preferably from 3:1 to 5:1, more preferably from 3.5:1 to 4.5:1, more preferably from 3.8:1 to 4.4:1, more preferably about 4:1. Depending on the hydrogenation potential of the hydrogenating agent the amount of hydrogenation agent may vary. In case of lithium aluminium hydride an amount of about 3:1 to 5:1, preferably 3.5:1 to 4.5:1, more preferably 4:1, turned out to produce good yields.

In another embodiment of the present invention, the conversion is performed in an aprotic solvent, preferably an aprotic apolar solvent. Suitable aprotic solvents are for example selected from the group consisting of diethyl ether, tetrahydrofuran, and dioxan.

In a preferred embodiment of the present invention, the solvent for the reduction step is diethyl ether, more preferably dry diethyl ether. Aprotic solvents such as diethyl ether turned out to give good yields for the reducing step according to the invention. Moreover, they have a good solubility for compounds according to formula (II), and thus, allow to easily separate the product after stopping the reaction with water.

The conversion is for example performed at a temperature in the range of from about 0°C to about 120°C. The reduction step can be performed at about the boiling temperature of the solvent (heating under reflux). Thus, depending on the solvent, the reduction step is preferably performed at a temperature in the range of from about 20°C to about 110°C, preferably about 30°C to about 100°C.

The conversion can be performed under normal pressure conditions. Typically, the reduction step is completed within several hours such as within about 2 h to about 24 h, preferably within about 3 h to about 18 h, more preferably within about 4 h to about 12 h, more preferably within about 4 h to about 6 h. However, it may be convenient to perform the reduction step over night (e.g. within about 8 h to about 18 h).

In a preferred embodiment of the present invention, the anion X⁻ is a halide anion, preferably selected from the group consisting of Cl⁻, Br⁻ or I⁻, more preferably I⁻.

The anion is preferably introduced during the preparation of the tetrahydropyridin-4-ylidene ammonium salt of formula (I).

In one embodiment of the present invention, the process may comprise further steps of preparing a tetrahydropyridin-4-ylidene ammonium salt of the general formula (I) prior to the reduction step. In the following, embodiments are shown for a compound of formula (I), wherein R^{1'}, R^{2'}, R^{3'}, R^{4'}, R^{5'}, R^{6'}, and R^{7'} are R¹, R², R³, R⁴, R⁵, R⁶, and R⁷, respectively. However, it will be appreciated that the embodiments similarly apply for a compound of formula (I) with R^{1'}, R^{2'}, R^{3'}, R^{4'}, R^{5'}, R^{6'}, and R^{7'}, wherein R^{1'}, R^{2'}, R^{3'}, R^{4'}, R^{5'}, R^{6'}, and R^{7'} are precursors of R¹, R², R³, R⁴, R⁵, R⁶, and R⁷, respectively, which are reducible to R¹, R², R³, R⁴, R⁵, R⁶, and R⁷, respectively.

Accordingly, the process in one embodiment includes one or more of the steps comprising
a) providing a compound of formula (III) 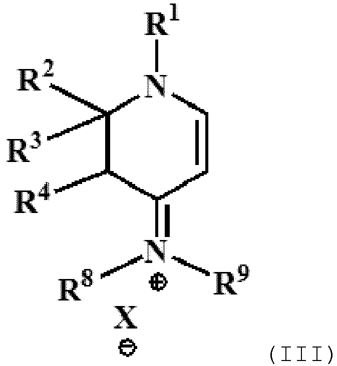 wherein a compound of formula (III) is a compound of formula (I), in which R⁵, R⁶ and R⁷ are hydrogen,
   optionally b) substituting a compound of formula (III) on ring position 3 using a halide of formula X¹-R⁵, for example under catalysis of potassium carbonate in chloroform, giving a compound of formula (IV): 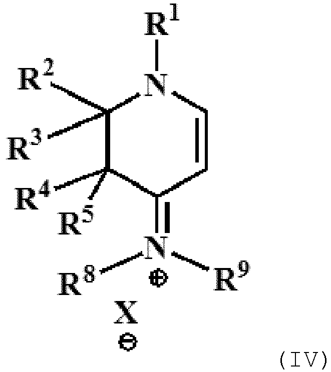 wherein a compound of formula (IV) is a compound of formula (I), in which R⁶ and R⁷ are hydrogen, preferably in this optional step b) said compound of formula (III) is provided with the provision that R¹ is not hydrogen or in the obtained compound of formula (IV) R¹ is identical to R⁵, optionally c) substituting of a compound with the formula (IV) or a compound of formula (III) on ring position 6 with a reaction introducing R⁷, which is not hydrogen, giving a compound of formula (V): 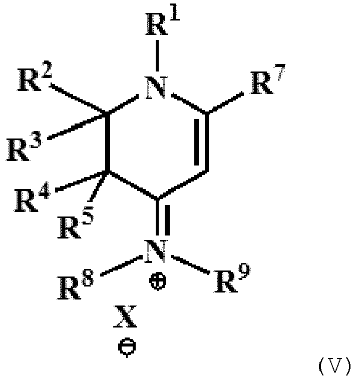 wherein a compound of formula (V) is a compound of formula (I), in which R⁶ is hydrogen
   optionally d) substituting a compound of formula (V) or a compound of formula (III) or a compound of formula (IV) on ring position 5 with a reaction introducing R⁶, which is not hydrogen, giving a compound of formula (I):

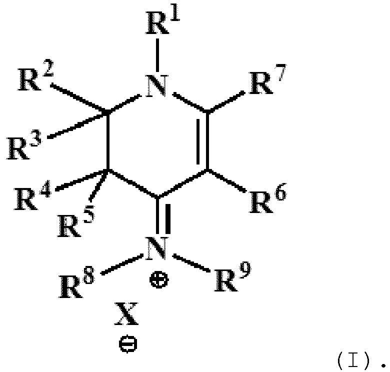

Two different approaches for preparing the tetrahydropyridin-4-ylidene ammonium salts, i.e. compounds according to formula (III) or (I), have been investigated and can be included in the process according to the invention. Both approaches start from α,β-unsaturated ketones and secondary amines (or their hydrothiocyanates or ammonium dialkyldithiocarbamates made thereof). α,β-Unsaturated ketones are commercial available or accessible by known procedures (e.g. H. G. O. Becker et al., 1986, Organikum, 16.Ed.,VEB Deutscher Verlag der Wissenschaften, P453). In situ generation of ammonium dialkyldithiocarbamates is described for example in K. Schweiger, 1980, Monatsh. Chem. 111:1175-1184.

In the first approach the piperidin-4-ones of the general formula (II) can be prepared from α,β- unsaturated ketones and hydrothiocyanates of secondary amines in a boiling solvent like toluene, dimethylformamide, bromobenzene, or xylene with water separation or without use of solvent giving dihydropyridin-2(1H)-thiones. They can be converted to their S-methyl salts by known procedures using methyliodide in chloroform at room temperature. A selective reduction of the S-methyl salt intermediate using deactivated Raney nickel in ethanol at room temperature yields a tetrahydropyridin-4-ylidene ammonium salt, wherein R¹, R⁵, R⁶, and R⁷ are hydrogen.

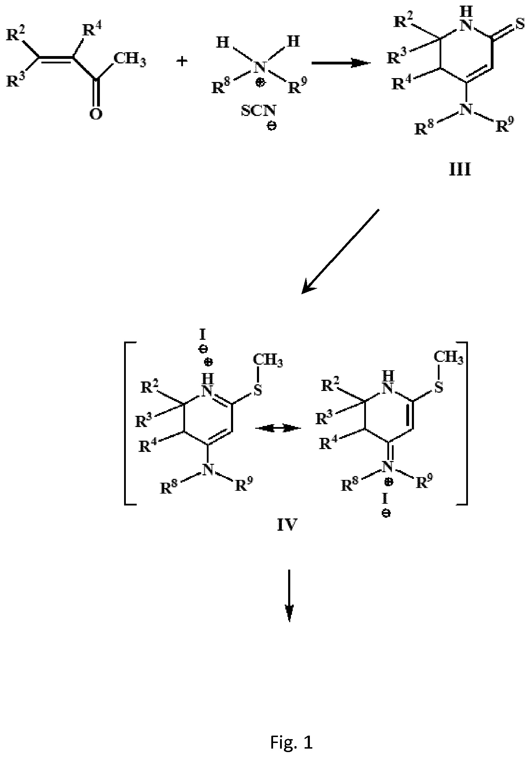

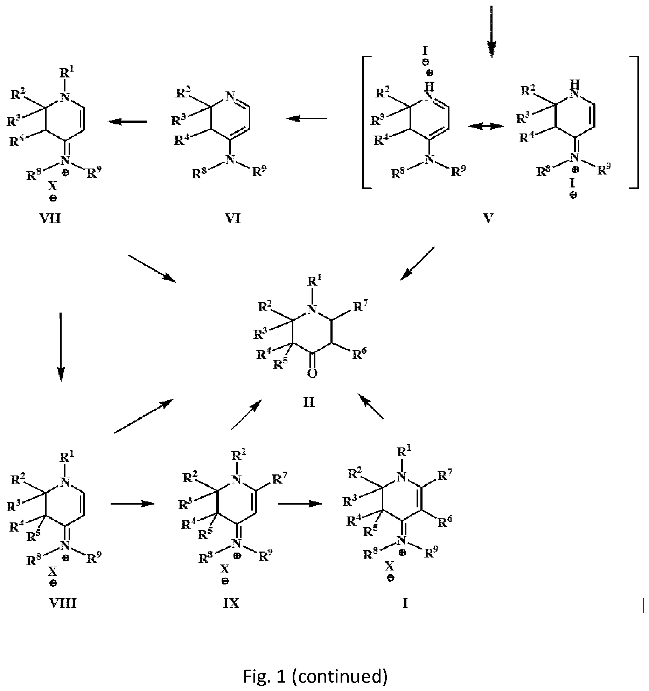

Optionally further substituents can be introduced. The tetrahydropyridin-4-ylidene ammonium salt can be converted to the free base by treatment with sodium hydroxide and extraction with diethyl ether, chloroform or dichloromethane. Subsequently, an N-alkylation leads to N-substituted compounds. Those compounds can be substituted in position 3 by treatment with halides (introduction of R⁵). Substitution in position 6 can be achieved to introduce an R⁷ different from hydrogen. Furthermore, position 5 can be substituted yielding compounds according to formula (I), wherein R⁶ is not hydrogen. The reactions of this first approach are summarized in the reaction scheme of Fig. 1.

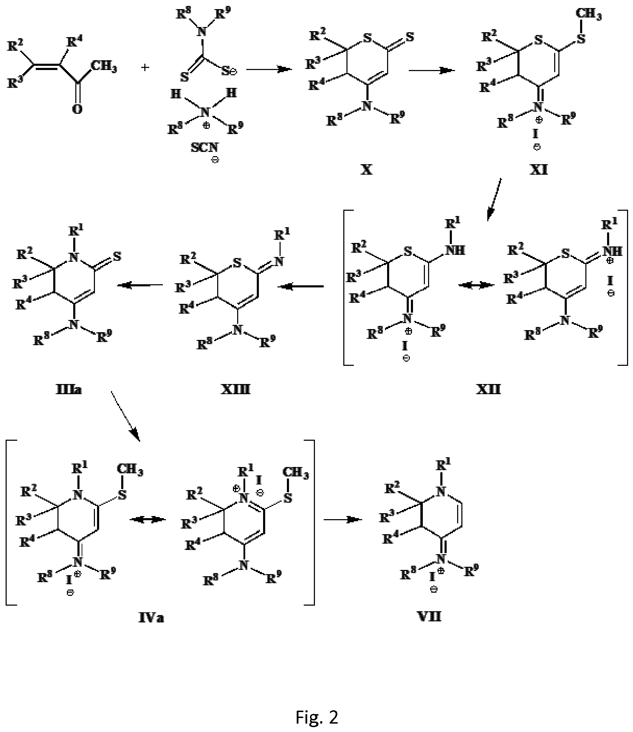

In the second approach the piperidin-4-ones of the general formula (II) can be prepared from α,β-unsaturated ketones and dialkylammonium dialkyldithiocarbamates in a boiling solvent like toluene, dimethylformamide, bromobenzene, or xylene with water separation or without use of solvent giving dialkylamino dihydrothiopyran-2-thiones. S-methylation yields in methylthio-4H-thiopyran-4-ylidene compounds. Subsequent aminolysis with primary amines leads to the iminium-enamin salts. Setting free the base of those compounds results in an amine and Dimroth rearrangement in boiling dimethyl formamide yields dihydropyridin-2(1H)-thiones. Those compounds can be treated as described above, performing an S-methylation to iodides and subsequently selective reducing the intermediates to tetrahydropyridin-4-ylidene ammonium salts. The reactions of this second approach are summarized in the reaction scheme of Fig. 2.

In a preferred embodiment of the present invention, the preparation of a compound according to formula (III) comprises
a) reacting a compound of the formula (VI): 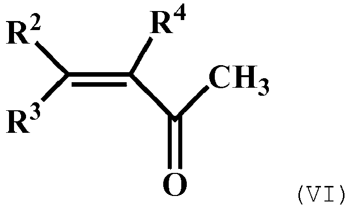 with a compound of formula (VII): 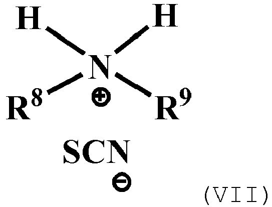 in a boiling organic solvent with or without water separation or without solvent, e.g. boiling in xylol to reflux at a water separator for about 6 h (Zigeuner, G. et al. Monatsh. Chem. 1976, 107, 1361-1367), to obtain a compound of formula (VIII): 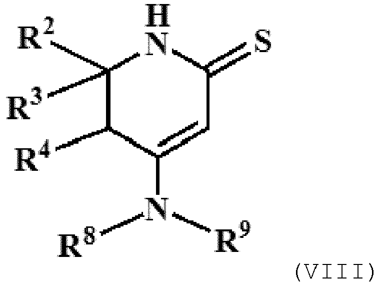
b) performing an S-methylation by dissolving the compound of formula (VIII) in chloroform and by treatment with methyl iodide to obtain a compound of formula (IX): 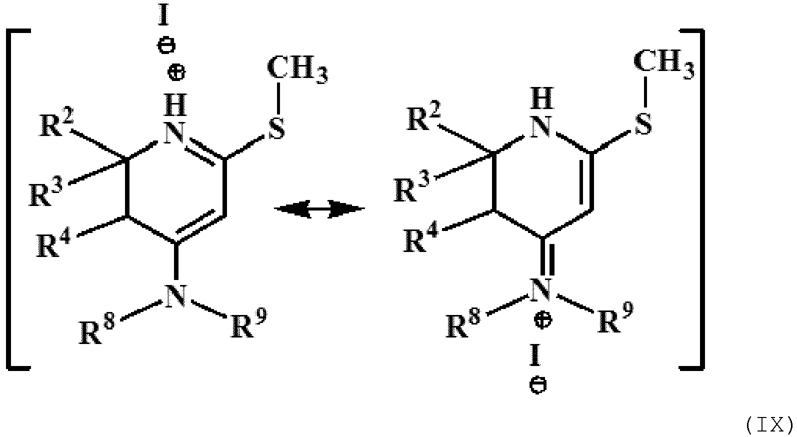
c) reducing a compound of formula (IX) using deactivated Raney nickel in ethanol to a compound of formula (X): 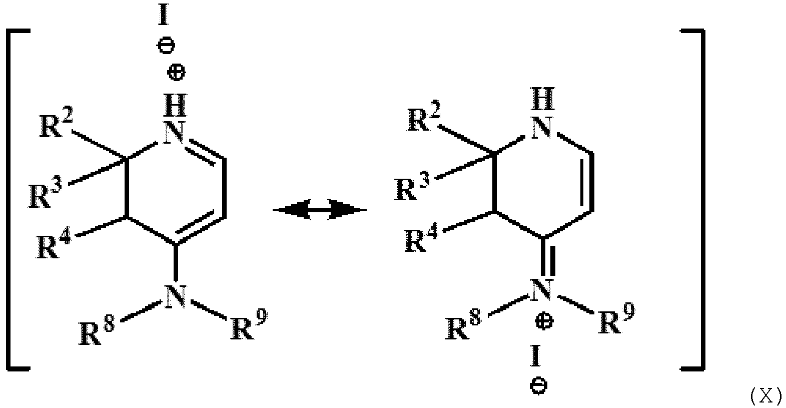 wherein a compound of formula (X) is a compound of formula (I), in which R¹, R⁵, R⁶ and R⁷ are hydrogen and X⁻ is I⁻ and
   optionally d) setting free the base corresponding base of the compound of formula (X) to yield a compound with the formula (XI): 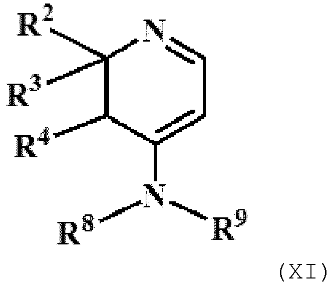 and substituting compounds of formula (XI) on the ring nitrogen by applying R¹-X, e.g. benzyl bromide as previously described (N. Mohsin et al. 2018 European Journal of Medicinal Chemistry 143: 97-106) to give a compound with the formula (III) 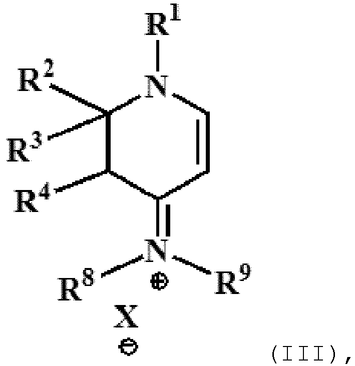
wherein a compound of formula (III) is a compound of formula (I), in which R⁵, R⁶ and R⁷ are hydrogen.

In an alternative embodiment of the present invention, the preparation of a compound according to formula (III) comprises
a) reacting compounds of the formula (VI) 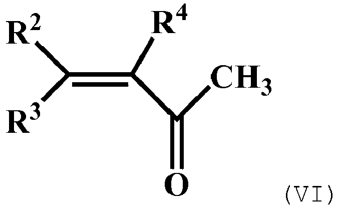 with a dialkylammonium dialkyldithiocarbamate of formula (XII) 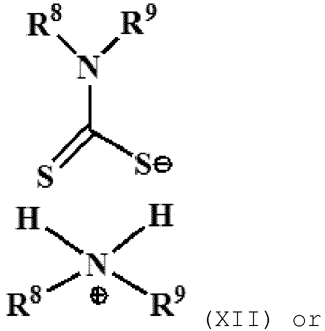 with a mixture of R₈-NH-R₉ and CS₂, which allows to obtain the dialkylammonium dialkyldithiocarbamate *in situ*, in a boiling organic solvent with or without water separation or without solvent to obtain a compound of formula (XIII) 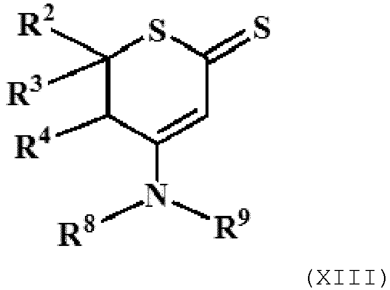
b) performing an S-methylation by dissolving the compound of formula (XIII) in chloroform and by treatment with methyl iodide to obtain a compound of formula (XIV) 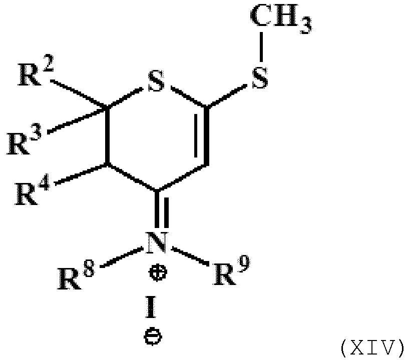
c) performing an aminolysis of the compound of formula (XIV) by using a primary amine NH₂R¹ giving a compound of formula (XV) 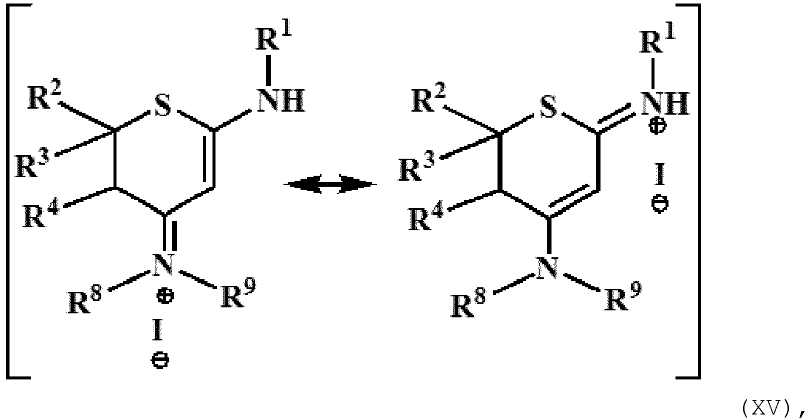
d) setting free the corresponding base of the compound of formula (XV) to obtain a compound with the formula (XVI) 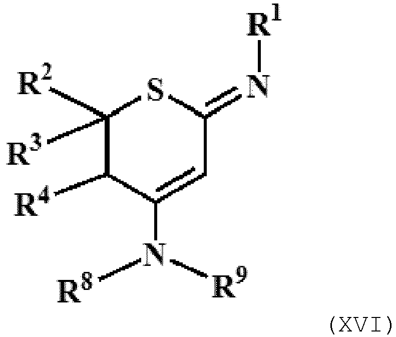
e) performing a Dimroth rearrangement with the compound of formula (XVI) to yield a compound of formula (XVII) 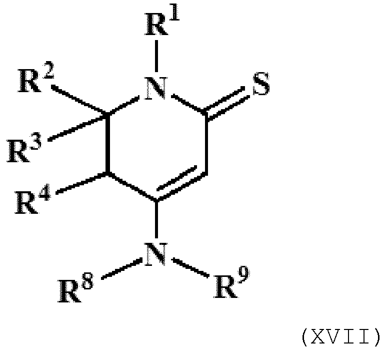 wherein preferably this step including a Dimrothrearrangement is performed as described before (Schweiger K. Monatsh. Chem. 1983, 114, 581-592.)
f) performing an S-methylation of the compound of formula (XVII) in chloroform by treatment with methyl iodide to yield a compound of formula (XVIII) 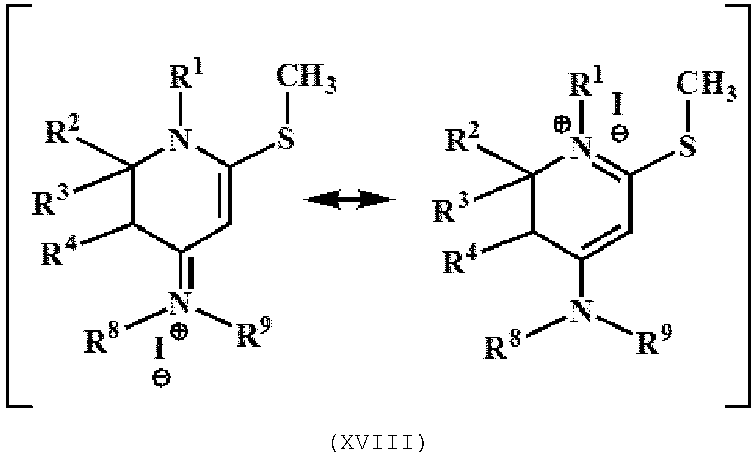
g) exchanging the anion I⁻ with another anion X⁻ or otherwise and preferably the anion X⁻ is I⁻,
h) reducing of the compound of formula (XVIII) using deactivated Raney nickel in ethanol to a compound of formula (III) 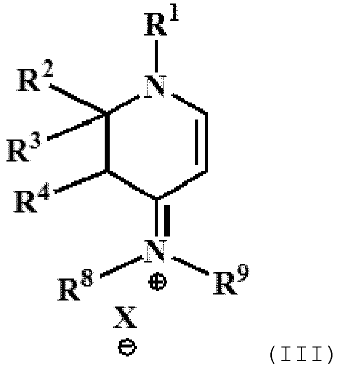 wherein a compound of formula (III) is a compound of formula (I), in which R⁵, R⁶ and R⁷ are hydrogen.

In the process according to the invention, R⁸ and R⁹ can be identical or can differ from each other.

In an embodiment of the present invention, R⁸ and R⁹ may together with the nitrogen form a non-aromatic heterocyclic ring. Preferably, the heterocyclic ring comprises 3 to 7 members. Moreover, the heterocyclic ring may be substituted and/or may contain in addition to the nitrogen one or more heteroatoms selected from the group consisting of nitrogen and oxygen.

In a particularly preferred embodiment of the present invention, R⁸ and R⁹ form together with the nitrogen to which they a bound form a heterocyclic ring, e.g., a 3 to 7 membered heterocyclic ring, for example selected from the group consisting of pyrrolidine, piperidine, morpholine, N-methylpiperazine, azepane, aziridine and azetidine, more preferably a 5 to 6 membered heterocyclic ring selected from the group consisting of pyrrolidine, piperidine, morpholine and N-methylpiperazine. The heterocyclic ring may be substituted with one or more substituents, wherein the substituent is preferably selected from C₁-C₁₀-alkyl and amino.

In the process of preparing a compound of formula (II) it is preferred that at least one of R², R^{2'}, R³, R^{3'}, R⁷, or R^{7'} is not hydrogen, preferably wherein either R² and R^{2'} or R³ and R^{3'} or all of R², R^{2'}, R³ and R^{3'} are not hydrogen. Also in the compound of formula (II) according to the present invention, at least one of R², R³, or R⁷ is not hydrogen, more preferably at least one of R² and R³ is not hydrogen. Compounds according to formula (II), wherein at least one of R², R³, or R⁷ is not hydrogen, are substituted at the position adjacent to the nitrogen (2-substituted piperidin-4-ones). Especially, the positions of R², R³, or R⁷ are not easily accessible with other processes. The process according to the invention allows substitution at the 2-position in the piperidine-ring and provides a piperidin-4-one compound of formula (II) according to the invention.

Also various other substitutions at the piperidine-ring are available and the following embodiments relating to the substituents R¹, R², R³, R⁴, R⁵, R⁶, and R⁷ apply for the process and the compound according to the invention.

The substitutions R^{1'}, R^{2'}, R^{3'}, R^{4'}, R^{5'}, R^{6'}, and R^{7'} tetrahydropyridin-4-ylidene ammonium salt of formula (I) preferably are identical to R¹, R², R³, R⁴, R⁵, R⁶, and R⁷, respectively. However, they may also be precursors of R¹, R², R³, R⁴, R⁵, R⁶, and R⁷, respectively, which are reducible to R¹, R², R³, R⁴, R⁵, R⁶, and R⁷, respectively. In the latter case, the person skilled in the art can easily derive the relation between the starting material and the reduced substituent R¹, R², R³, R⁴, R⁵, R⁶, and R⁷. Exemplarily, it is referred to the example 5 herein, wherein an R^{1'} is (ethoxycarbonyl)methyl and reduced to R¹ being 2-hydroxyethyl.

In the process or compound according to the present invention, it is further preferred that any substituent b) to 1) as previously defined and independently to each other is unsubstituted or substituted with one or more further substituent(s) selected from the group consisting of halogenide, cyano, hydroxyl, C₁-C₄-alkoxy, and C₁-C₄-alkyl.

The term "substituted" refers to a chemical entity being substituted with one or more substituents. The term "optionally substituted" relating to a chemical entity means that the chemical entity may be substituted or not, i.e. both situations are included. The term "one or more substituents" refers to from one to the maximum number of substituents possible, depending on the number of available substitution sites.

The term "alkyl" as used herein refers to straight or branched hydrocarbon chains having a specified number of carbon atoms. For example, C₁-C₁₀-alkyl refers to a straight or branched alkyl group having at least 1 and at most 10 carbon atoms. Alkyl groups having a straight chain preferably represent C₁-C₇alkyl. Useful branched chains, which preferably represent C₃-C₁₀alkyl, are for example isopropyl, isobutyl, sec. butyl, tert. butyl, pentan-1-yl, pentan-2-yl, pentan-3-yl and 6-ethyloctan-2-yl groups.

The term "alkyl being substituted with a substituent containing a nitrogen atom" as used herein refers to an "alkyl" as defined above bearing an additional nitrogen-containing substituent. The substituent containing a nitrogen atom is preferably selected from primary, secondary and tertiary amines, and cyano. Useful examples of alkyl groups which are substituted with a substituent containing a nitrogen atom include for example diethylaminoethyl, diethylaminopropyl, diethylaminobutyl groups as well as dipropylaminoethyl, dipropylaminopropyl, dipropylaminobutyl, diisopropylaminoethyl, diisopropylaminopropyl, diisopropylaminobutyl, dibutylaminoethyl, dibutylaminopropyl and dibutylaminobutyl moieties.

The term "alkyl being substituted with a non-aromatic heterocyclic ring" as used herein refers to an alkyl as defined above being substituted with a monocyclic preferably 3-7-membered saturated hydrocarbon ring containing at least one heteroatom independently selected from oxygen and nitrogen. The heterocyclic ring may be substituted, wherein preferred substituents are selected from methyl or hydroxy. Useful examples of alkyl residues being substituted with a non-aromatic heterocyclic ring include, but are not limited to pyrrolidinoethyl, pyrrolidinopropyl, pyrrolidinobutyl, piperdinoethyl, piperidinopropyl, piperidinobutyl, 4-methylpiperazinoethyl, 4-methylpiperazinopropyl, 4-methylpiperazinobutyl, 4-hydroxypiperidinoethyl, 4-hydroxypiperidinopropyl, 4-hydroxypiperidinobutyl, azepan-1-ylethyl, azepan-1-ylpropyl, azepan-1-ylbutyl, aziridin-1-ylethyl, aziridin-1-ylpropyl, aziridin-1-ylbutyl, azetidin-1-ylethyl, azetidin-1-ylpropyl and azetidin-1-ylbutyl residues.

The term "aryl" as used herein refers to an aromatic carbocyclic ring having a specified number of carbon atoms. For example, C₅-C₆-aryl refers to an aromatic ring having at least 5 and at most 6 carbon atoms. The term "aryl" also refers to a multicyclic group having more than one aromatic ring, such as C₁₀-C₁₄-aryl. Useful examples are cyclopentadienyl, phenyl, naphthyl, anthracyl, phenantryl. Typical substituted aryl residues are 4-chlorophenyl, 4-bromophenyl, 4-fluorophenyl, 4-trifluoromethylphenyl, 4-methylphenyl, 4-methoxyphenyl, 4-isopropylphenyl, 6-fluoro-naphthalen-1-yl, 6-chloro-naphtalen-1-yl, 6-methoxy-naphthalen-1-yl, 6-trifluoromethyl-naphthalen-1-yl, 3-methoxy-1-naphthalen-1-yl, 3-chloro-naphthalen-1-yl, 3-trifluoromethyl-naphthalen-1-yl, 3-fluoro-naphthalen-1-yl, 3,5-dichlorophenyl, 3,4-dichlorophenyl, 2-chlorophenyl, 2,6-dichlorophenyl, 3,5-difluoro-naphthalen-1-yl, 3,5-dichloro-naphthalen-1-yl, 3,5-dimethoxy-naphthalen-1-yl, 3,5-bis(trifluoromethyl)naphthalen-1-yl, 4-nitrophenyl, and 4-aminophenyl.

The term "heteroaryl" as used herein refers to aromatic groups having one or more heteroatoms (O, S or N). A multicyclic group having one or more heteroatoms, wherein at least one ring of the group is heteroaromatic, is referred to as a "heteroaryl" as well. Typical substituted heteroaryl residues are 2-methylpyridin-4-yl, 3-methylpyridin-4-yl, 4-methylpyridin-3-yl, 5-methylpyridin-3-yl, 6-methylpyridin-3-yl, 2-methylpyridin-3-yl, 6-chloro-quinolin-1-yl, 6-fluoroquinolin-1-yl, 6-methoxy-quinolin-1-yl, 6-trifluoromethylquinolin-1-yl, 7-chloro-quinolin-1-yl, 7-fluoro-quinolin-1-yl, 7-methoxy-quinolin-1-yl, 7-trifluoromethyl-quinolin-1-yl, 8-chloro-quinolin-1-yl, 8-fluoro-quinolin-1-yl, 8-methoxyquinolin-1-yl, 8-trifluoromethyl-quinolin-1-yl substituents.

Useful aryl alkyl groups, i.e. "C₅-C₁₄-aryl-C₁-C₄-alkyl", wherein the aryl preferably is a C₆-C₁₄-aryl and the alkyl is preferably a C₁-C₄alkyl, include for example benzyl, naphthalen-1-yl-methyl, naphthalen-2-yl-methyl, cyclopentadienyl-methyl, cycloheptadienyl-methyl, anthracene-methyl, and phenanthrene-methyl groups. Furthermore, phenylethyl, phenylpropyl and phenylbutyl groups are considered.

Useful heteroaryl alkyl"groups, i.e. "heteroaryl-C₁-C₄-alkyl", wherein the heteroaryl preferably is selected from a 5 to 10 membered heterocyclic ring and alkyl preferably represents C₁-C₄-alkyl, include for example pyridin-4-ylmethyl, pyridin-3-ylmethyl, pyridin-2-ylmethyl, pyridin-4-ylpropyl, pyridin-4-ylbutyl, pyrrol-2-ylmethyl, pyrrol-3-ylmethyl, indole-3-ylmethyl, indol-3-ylethyl and ferrocene-methyl substituents.

For any aryl or heteroaryl group, C₆-C₁₀-aryl and 5 to 10 membered heteroaryl, respectively, are preferred embodiments. The aryl or heteroaryl group may be substituted. Preferred substituents are selected from the group consisting of halogen, nitro, amino, alkyl or alkoxy.

The substituents R¹, R², R³, and R⁴ are introduced during synthesis of the tetrahydropyridin-4-ylidene ammonium salt. Above, it is also shown how substitutions R⁵, R⁶, and R⁷ can be introduced at the other sites of the piperidin ring prior before the reduction step. In another embodiment, at least one of R⁵, R⁶, and R⁷ is hydrogen, preferably, all of R⁵, R⁶, and R⁷ are hydrogen.

In another preferred embodiment of the present invention, R¹, R², R³, R⁴, R⁵, R⁶, R⁷, are independently from each other selected from the group consisting of a) to f). These substituents show a low susceptibility towards modifications by reduction and thus, they are very well suitable in the process according to the invention.

More preferably, R¹, R², R³, R⁴ independently from each other are selected from the group consisting of hydrogen, C₁-C₆-alkyl, C₆-aryl, heteroaryl, C₆-aryl-C₁-C₄-alkyl and heteroaryl-C₁-C₄-alkyl and R⁵, R⁶, and R⁷ are hydrogen.

In another embodiment of the process or compound according to the present invention, R⁴ is selected from the group consisting of hydrogen and benzyl.

In a preferred embodiment of the present invention, R² and R³ both are methyl. In this embodiment, R¹ is preferably selected from the group consisting of prop-2-yl, 1-methyl-2-phenethyl, 2-hydroxyethyl, 2-hydroxypropyl, 2-(1H-indol-3-yl)ethyl, pyridin-2-ylmethyl, 3-cyano-3-diphenylpropyl, 4-oxo-4-(4-fluorphenyl)butyl, 4-di(4-fluorphenyl)butyl, 3-(4-fluorphenoxy)propyl, 2-(4-methoxyphenyl)ethyl, 3-(4-fluorphenoxy)-2-hydroxylpropyl, and 3-(2-oxo-1-benzimidazolinyl)propyl, preferably 2-(1H-indol-3-yl)ethyl. If R¹ is selected from the group consisting of hydrogen, methyl, isopropyl, n-butyl, benzyl, phenyl, 2-phenethyl, allyl, acetyl, methylsulfonyl, benzoyl and benzyl carboxylate, then at least one of R⁴, R⁵, R⁶, and R⁷ is not hydrogen, preferably, at least R⁴ is not hydrogen, e.g. R⁴ is benzyl. If all of R⁴, R⁵, R⁶, and R⁷ are hydrogen, then R¹ is not selected from the group consisting of hydrogen, methyl, isopropyl, n-butyl, benzyl, phenyl, 2-phenethyl, allyl, acetyl, methylsulfonyl, benzoyl and benzyl carboxylate.

In another preferred embodiment of the present invention, R² or R³ is isopropyl and the other one of R² or R³ is hydrogen. In this embodiment, preferably, R¹ is selected from the group consisting of prop-2-yl, 2-phenethyl, 1-methyl-2-phenethyl, 2-hydroxyethyl, 2-hydroxypropyl, 2-(1H-indol-3-yl)ethyl, pyridin-2-ylmethyl, 3-cyano-3-diphenylpropyl-, 4-oxo-4-(4-fluorphenyl)butyl, 4-di(4-fluorphenyl)butyl, 3-(4-fluorphenoxy)propyl, 2-(4-methoxyphenyl)ethyl, 3-(4-fluorphenoxy)-2-hydroxylpropyl, and 3-(2-oxo-1-benzimidazolinyl)propyl, preferably 2-phenethyl, 2-(1H-indol-3-yl)ethyl. If R¹ is selected from the group consisting of hydrogen, methyl, isobutyl, benzyl, phenyl, acetyl, methyl carboxylate, tert-butyl carboxylate, phenyl carboxylate and benzyl carboxylate, then at least one of R⁴, R⁵, R⁶, and R⁷ is not hydrogen, preferably, at least R⁴ is not hydrogen, e.g. R⁴ is benzyl. If all of R⁴, R⁵, R⁶, and R⁷ are hydrogen, then R¹ is not selected from the group consisting of prop-2-yl, 2-phenethyl, 1-methyl-2-phenethyl, 2-hydroxyethyl, 2-hydroxypropyl, 2-(1H-indol-3-yl)ethyl, pyridin-2-ylmethyl, 3-cyano-3-diphenylpropyl-, 4-oxo-4-(4-fluorphenyl)butyl, 4-di(4-fluorphenyl)butyl, 3-(4-fluorphenoxy)propyl, 2-(4-methoxyphenyl)ethyl, 3-(4-fluorphenoxy)-2-hydroxylpropyl, and 3-(2-oxo-1-benzimidazolinyl)propyl, preferably 2-phenethyl, 2-(1H-indol-3-yl)ethyl.

Additionally, and independent from R¹, in an embodiment of the present invention R⁴ is selected from the group consisting of benzyl and hydrogen, preferably benzyl.

In a specific embodiment of the present invention the compound of formula (II) is selected from the group consisting of (2RS)-(±)-2-isopropyl-1-phenethylpiperidin-4-one, (2RS)-(±)-2-isopropyl-1-propylpiperidin-4-one, (2RS)-(±)-1-butyl-2-isopropylpiperidin-4-one, (2RS)-(±)-1-sec-butyl-2-isopropylpiperidin-4-one, (2RS)-(±)-1-(2-hydroxyethyl)-2-isopropylpiperidin-4-one, (2RS)-(±)-1-(2-hydroxypropyl)-2-isopropylpiperidin-4-one, (2RS)-(±)-2-isopropyl-1-vinylpiperidin-4-one, (2RS)-(±)-1-allyl-2-isopropylpiperidin-4-one, (2RS)-(±)-1-cyclohexyl-2-isopropylpiperidin-4-one, (2RS)-(±)-1-(cyclohexylmethyl)-2-isopropylpiperidin-4-one, 2,2-dimethyl-1-vinylpiperidin-4-one, 1-(2-hydroxyethyl)-2,2-dimethylpiperidin-4-one, and 1-(2-hydroxypropyl)-2,2-dimethylpiperidin-4-one, preferably selected from the group consisting of (2RS)-(±)-2-isopropyl-1-phenethylpiperidin-4-one, (2RS)-(±)-2-isopropyl-1-propylpiperidin-4-one, (2RS)-(±)-1-butyl-2-isopropylpiperidin-4-one, (2RS)-(±)-1-sec-butyl-2-isopropylpiperidin-4-one, (2RS)-(±)-1-(2-hydroxyethyl)-2-isopropylpiperidin-4-one, (2RS)-(±)-1-(2-hydroxypropyl)-2-isopropylpiperidin-4-one, (2RS)-(±)-1-cyclohexyl-2-isopropylpiperidin-4-one, (2RS)-(±)-1-(cyclohexylmethyl)-2-isopropylpiperidin-4-one, 1-(2-hydroxyethyl)-2,2-dimethylpiperidin-4-one, and 1-(2-hydroxypropyl)-2,2-dimethylpiperidin-4-one, more preferably selected from the group consisting of (2RS)-(±)-2-isopropyl-1-(2-phenylethyl)piperidine-4-one, (3RS)-(±)-1,3-dibenzyl-2,2-dimethylpiperidin-4-one, (2RS, 3RS)-(±)-3-benzyl-2-isopropyl-1-(2-phenylethyl)piperidin-4-one, (2RS, 3SR)-(±)-3-benzyl-2-isopropyl-1-(2-phenylethyl)piperidin-4-one, (3RS)-(±)-3-benzyl-1,2,2-trimethylpiperidin-4-one, and 1-(2-hydroxyethyl)-2,2-dimethylpiperidin-4-one.

Certain tetrahydropyridinylidene ammonium salts of the general formula (I) and some of the piperidin-4-ones of general formula (II) may exist in different stereoisomeric forms, wherein the individual stereoisomers, including enantiomers and diastereoisomers, and mixtures thereof are also within the scope of the present invention. Individual isomers can be obtained by methods known to those skilled in the art. Individual isomers and mixtures thereof as well as racemic mixtures (as indicated by the prefix "(RS)-") are included as well.

Another aspect of the present invention relates to a method of derivatizing a piperidin-4-one, which results in a valuable derivatizing product.

The method of derivatizing a piperidin-4-one may comprise the process according to the invention (preparing a piperidin-4-one comprising a step of reducing a tetrahydropyridin-4-ylidene ammonium salt to a compound of formula (II)) and a further step of reacting the compound of formula (II).

In another embodiment of the present invention, the method of derivatizing a piperidin-4-one only includes providing the compound of formula (II) according to the invention as defined above.

Derivatizing products of piperidin-4-ones are 4-amino-piperidines, i.e. compounds of formula (XIX), 4-alkoxypiperidines, i.e. compounds of formula (XX), and 4-aryl-4-hydroxypiperidines, i.e. compounds of formula (XXI) as shown in the following reaction scheme: 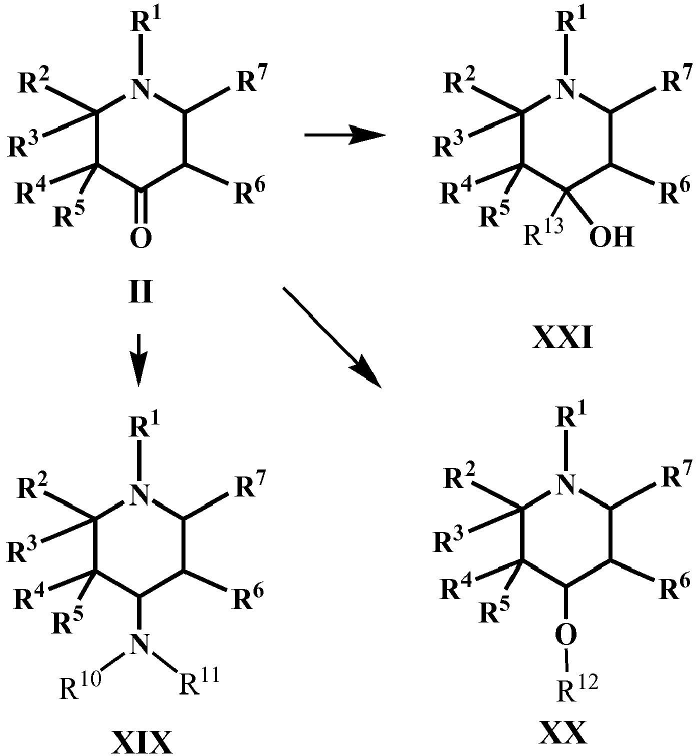 wherein R¹⁰, R¹¹, R¹² and R¹³ are independently of each other hydrogen or an organic moiety.

Derivatizing products relate to important drug substances. 4-Amino-piperidines are for example related to fentanyl, an anesthetic drug, indoramin, an antiadrenergic agent, and bamipine, an antihistaminic agent. 4-Aryl-4-hydroxypiperidines include for example the antipsychotic drug haloperidol.

In an embodiment of the present invention, the method of derivatizing includes a reductive amination to obtain a compound according to formula (XIX). In a preferred embodiment, the compound according to formula (XIX) is a derivative, preferably a 2-substituted derivative (i.e. wherein at least one of R², R³, or R⁷ is not hydrogen) of a drug compound selected from the group of astemizole, bamipine, benperidol, bezitramide, cisapride, clebopride, domperidone, enzastaurin, fentanyl, indoramin, lorcainide, α-methylfentanyl, pimozide, sabeluzole, and timiperone.

In a preferred embodiment of the present invention, R¹⁰, R¹¹, R¹² and R¹³ are derived from the drug compound, to which the derivatizing product is related. In general, the term "organic moiety" as used herein refers to R¹⁰, R¹¹, R¹² and R¹³ being selected from the group consisting of C₁-C₁₀-alkyl, C₆-C₁₄-aryl, heteroaryl, wherein the heteroaryl is 5 to 14 membered aromatic ring system with at least one heteroatom selected from the group consisting of N, O, and S atoms, C₅-C₁₄-aryl-C₁-C₄-alkyl, heteroaryl-C₁-C₄-alkyl, C₂-C₁₀-alkenyl containing one or more double bonds, C₂-C₁₀-alkinyl containing one or more triple bonds, C₅-C₁₄-aryl-C₂-C₄-alkenyl containing one or more double bonds, C₅-C₁₄-aryl-C₂-C₄-alkinyl containing one or more triple bonds, heteroaryl-C₂-C₄-alkenyl containing one or more double bonds, and heteroaryl-C₂-C₄-alkinyl containing one or more triple bonds, wherein the heteroaryl is 5 to 14 membered aromatic ring system with at least one heteroatom selected from the group consisting of N, O, and S atoms, or in case of R¹⁰ and R¹¹, the organic moiety is made in that R¹⁰ and R¹¹ form together with the nitrogen to which they are bound a 3 to 7 membered unsubstituted or substituted non-aromatic heterocyclic ring, wherein the non-aromatic heterocyclic ring optionally contains one or more additional heteroatoms selected from the group consisting of N, O, and S atoms. Optionally, one or more of R¹⁰, R¹¹, R¹² and R¹³ being an organic moiety as defined above are further substituted. Preferably, the organic moiety as previously defined is unsubstituted or substituted with one or more further substituent(s) selected from the group consisting of halogenide, cyano, oxo, amino, thio, hydroxyl, C₁-C₄-acyl, C₁-C₄-alkoxy, and C₁-C₄-alkyl.

In a preferred embodiment of the present invention, the method of derivatizing a compound of formula (II) according to the invention or obtained by a process according to the invention includes a reductive amination and the compound according to formula (XIX) is N-(2,2-Dimethyl-1-phenethylpiperidin-4-yl)-N-phenylpropionamid.

The following reaction scheme shows the derivatizing method to obtain the novel 2-substituted derivative of fentanyl (2,2-dimethyl fentanyl), wherein (4R,S)-N-(2,2-dimethyl-1-phenethylpiperidin-4-yl)-N-phenylpropionamide was prepared from 2,2-dimethyl-1-phenethylpiperidine-4-one via a reductive amination with aniline to (4R,S)-2,2-dimethyl-1-phenethyl-N-phenylpiperidin-4-amine and subsequent formation to an amide using propionylchloride to 2,2-dimethyl fentanyl:

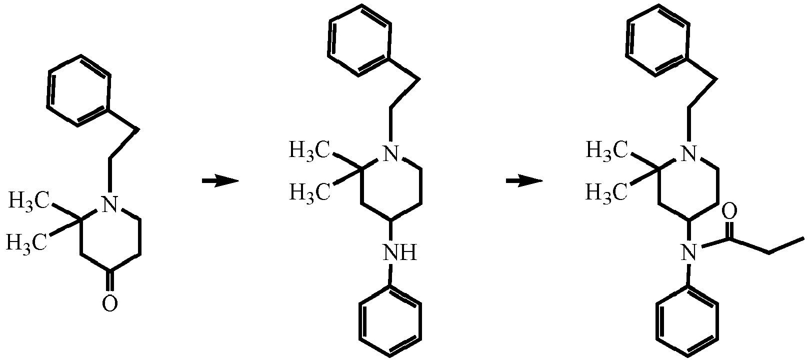

### EXAMPLES

The present invention is further demonstrated and illustrated by following examples, yet without being restricted thereto.

### Example 1: (2RS)-(±)-2-Isopropyl-1-phenethylpiperidine-4-one

### (6RS)-(±)-4-Dimethylamino-5,6-dihydro-6-isopropylpyridine-2(1H)-thione:

5-methylhex-3-en-2-one (22.8 g, 0.2 mol) and dimethylammonium thiocyanate (10.4 g, 0.1 mol) were suspended in 100 ml of bromobenzene. The mixture was reluxed for 8 h at a water separator and the solvent removed in vacuo. The residue was triturated with propan-2-ol, filtered with suction and recrystallized. Yield: 13.53 g (68%); M.p.: 142°C.

### (2RS)-(±)-N-(2-Isopropyl-6-methysulfanyl-1,2,3,4-tetrahydropyridin-4-yliden)-N,N-dimethylammoniumiodide:

(6RS)-(±)-4-Dimethylamino-5,6-dihydro-6-isopropylpyridine-2(*1H*)-thione (19.8 g, 0.1 mol) was dissolved in 50 ml of chloroform. Upon cooling and stirring on an ice bath, methyl iodide (17 g, 0.12 mol) dissolved in 50 ml of chloroform were dropped to the solution during 20 min. The ice bath was removed and the mixture stirred overnight at room temperature. Then the solvent was evaporated in vacuo and the residue recrystallized from chloroform/ethyl acetate. Yield: 33.4 g (98%); M.p.: 145°C.

### General procedure for the preparation of deactivated raney nickel and the selective reduction process:

Powdered nickel/aluminium alloy was given into a big beaker, water was added and solid NaOH was added cautiosly in that way, that the reaction gets not out of control. After the main reaction ceased, the beaker was put into a water bath at 70°C for 30 min. After that, the liquid was decanted and the solid nickel was washed with water until the solution reacted neutral (20 times). Then it was washed twice with ethanol and given to a solution of the methylthio compound in the minimum amount of ethanol that is needed for dissolution of the starting material, and stirred at room temperature. The catalyst was removed by suction and washed with ethanol. The filtrate and washings were combined and evaporated in vacuo. The residue was dissolved in chloroform and filtered and the solvent evaporated in vacuo giving the reduction products as nearly pure green resins, which were further purified.

### (2RS)-(±)-N-(2-Isopropyl-1,2,3,4-tetrahydropyridin-4-yliden)-N,N-dimethylammoniumiodide:

(2RS)-(±)-N-(2-Isopropyl-6-methylsulfanyl-1,2,3,4-tetrahydropyridin-4-yliden)-N,N-dimethylammoniumiodide (25 g, 73.5 mmol) dissolved in ethanol (150 ml) was treated with raney nickel from 75 g nickel/aluminium alloy and 150 g of caustic soda for 45 min. in a total volume of 300 ml ethanol. The residue was recrystallized from chloroform/ethyl acetate giving a beige precipitate. Yield: 14.2 g (65%). M.p.: 178°C; ¹H NMR (DMSO-d₆, *δ*, 400 MHz): 0.94 (t, *J* = 6.4 Hz, 6H, CH(C*H*₃)₂), 1.96 (sex, *J* = 6.6 Hz, 1H, C*H*(CH₃), 2.60 (dd, *J* = 16.9, 13.9 Hz, 1H, 3-H), 2.86 (dd, *J* = 17.2, 6.2 Hz, 1H, 3-H), 3.16, 3.25 (2s, 6H, N(CH₃)₂), 3.46 - 3.52 (m, 1H, 2-H), 5.19 (d, *J* = 6.6 Hz, 1H, 5-H), 7.69 (d, *J* = 6.6 Hz, 1H, 6-H), 9.24 (br, s, 1H, 1-H) ppm.

### (2RS)-(±)-N-(2-Isopropyl-1-(2-phenylethyl)-1,2,3,4-tetrahydropyridin-4-yliden)-N,N-dimethylammoniumbromide:

(2RS)-(±)-N-(2-Isopropyl-1,2,3,4-tetrahydropyridin-4-yliden)-N,N-dimethylammoniumiodide (14 g, 48.1 mmol) was stirred for half an hour with 2N NaOH and the mixture extracted exhaustively with dichloromethane. The combined organic layers were dried over sodium sulfate, filtered and the solvent evaporated giving (2RS)-(±)-2,3-dihydro-2-isopropyl-N,N-dimethylpyridin-4-amine (8 g, 48.1 mmol). This was dissolved in 60 ml of chloroform. Potassium carbonat (11.5 g, 83 mmol) was added and a solution of 2-phenylethylbromide (20 g, 109 mmol) in 60 ml of chloroform was added dropwise. The mixture was stirred for two weeks and then filtered and the solvent evaporated in vacuo. A part of the excess of 2-phenylethylbromid was distilled off on a boiling water bath in vacuo. The residue was dissolved in chloroform and ethyl acetate was added till the solution becomes turbid. The product crystallized upon stirring and cooling. Yield: 8.64 g (48%); M.p. 198°C, ¹H NMR (DMSO-d₆, *δ*, 400 MHz): 0.80 (d, *J* = 7.0 Hz, 3H, CH(C*H*₃)₂), 0.92 (d, *J* = 7.0 Hz, 3H, CH(C*H*₃)₂), 2.10 (sex, *J* = 6.6 Hz, 1H, C*H*(CH₃), 2.74 (dd, *J* = 18.0, 8.1 Hz, 1H, 3-H), 2.86 - 3.01 (m, 3H, 3-H, ArCH₂), 3.11, 3.26 (2s, 6H, N(CH₃)₂), 3.52 - 3.56 (m, 1H, 2-H), 3.66 (ddd, *J* = 13.6, 7.3, 5.9 Hz, 1H, NCH₂), 3.80 (ddd, *J* = 13.9, 7.7, 7.3 Hz, 1H, NCH₂), 5.07 (d, *J* = 7.0 Hz, 1H, 5-H), 7.21 - 7.33 (m, 5H, ArH), 7.53 (d, *J* = 6.6 Hz, 1H, 6-H) ppm.

### (2RS)-(±)-2-Isopropyl-1-(2-phenethyl)piperidine-4-one:

(2RS)-(±)-N-(2-Isopropyl-1-(2-phenylethyl)-1,2,3,4-tetrahydropyridin-4-yliden)-N,N-dimethylammoniumiodide (2.0 g, 5.69 mmol) was suspended in 180 ml of dry diethyl ether, the suspension was cooled on an ice bath and LiAlH₄ (903 mg, 23.8 mmol) was added cautiously. Then the mixture was refluxed overnight, cooled on an ice bath and water added cautiously via a dropping funnel which was fixed on the top of the reflux condenser. After the reaction was quenched completely, NaOH 2N was added and the reaction mixture transferred into a separatory funnel. The organic layer was separated and the aqueous phase extracted once with dichloromethane and twice with diethyl ether, all organic layers were combined, washed once with 2N NaOH and two times with brine and then dried over sodium sulfate. After filtration, the solvents were evaporated in vacuo and the residue subjected to column chromatography over silica gel using CH₂Cl₂:CH₃OH=20:1 as eluent. The fractions containing the pure product were combined and the solvent evaporated, giving 385 mg (28%) of an orange oil. ¹H NMR (CDCl₃, *δ*, 400 MHz): 0.88 (d, *J* = 7.3 Hz, 3H, CH₃), 0.90 (d, *J* = 7.0 Hz, 3H, CH₃), 1.87 (dsept, *J* = 6.6, 6.6 Hz, 1H, C*H*(CH₃)₂), 2.29 (dd, *J* = 13.7, 7.5 Hz, 1H, 3-H), 2.37 - 2.44 (m, 3H, 3-H, 5-H), 2.56 - 2.64 (m, 1H, 2-H), 2.79 - 2.95 (m, 5H, 6-H, ArCH₂, NCH₂), 3.21 - 3.27 (m, 1H, 6-H), 7.19 - 7.31 (m, 5H, ArH) ppm.

### Example 2: (3RS)-(±)-1,3-Dibenzyl-2,2-dimethylpiperidin-4-one

### 5,6-Dihydro-6,6-dimethyl-4-(pyrrolidin-1-yl)pyridin-2(1H)-thione:

A mixture of pyrrolidinium thiocyanate (54.6 g, 0.45 mol) and mesityloxide (90 g, 0.9 mol) in 350 ml of bromobenzene was refluxed over a water separator for 4h. Some solvent was removed by evaporation in vacuo. The resulting solution was left in the refrigerator and the product crystallized overnight. It was recrystallized from 2-propanol as green needles. Yield: 19 g (20%). M.p.: 254°C.

### N-(2,2-Dimethyl-6-methylsulfanyl-1,2,3,4-tetrahydropyridin-4-yliden)pyrrolidiniumiodide:

5,6-Dihydro-6,6-dimethyl-4-(pyrrolidin-1-yl)pyridin-2(*1H*)-thione (17.0 g, 80 mmol) reacted in 50 ml of chloroform with methyl iodide (13.6 g, 96 mmol) in the usual manner. Yield: 23.2 g (83 %). M.p.: 160°C.

### N-(2,2-Dimethyl-1,2,3,4-tetrahydropyridin-4-yliden)pyrrolidiniumiodide:

N-(2,2-Dimethyl-6-methylsulfanyl-1,2,3,4-tetrahydropyridin-4-yliden)pyrrolidiniumiodide (36 g, 0.1 mol) was treated with raney nickel prepared from 100 g nickel/aluminium alloy and 200 g of caustic soda in 420 ml total volume of ethanol giving 23 g (73%) as yellow needles. M.p. 192°C, ¹H NMR (DMSO-d₆, *δ*, 400 MHz): 1.27 (s, 6H, 2CH₃), 1.91 - 1.97 (m, 4H, (CH₂)₂), 2.84 (s, 2H, 3-H), 3.49 (t, *J* = 6.2 Hz, 2H, NCH₂), 3.66 (t, *J* = 6.2 Hz, 2H, NCH₂), 5.09 (d, *J* = 6.6 Hz, 1H, 5-H), 7.58 (d, *J* = 6.6 Hz, 1H, 6-H), 9.27 (br, s, 1H, 1-H) ppm.

### (3RS)-(±)-N-(1,3-Dibenzyl-2,2-dimethyl-1,2,3,4-tetrahydropyridin-4-ylidene)pyrrolidinium bromide:

N-(2,2-Dimethyl-1,2,3,4-tetrahydropyridin-4-yliden)pyrrolidiniumiodide (660 mg, 2.16 mmol) was stirred for half an hour with 2N NaOH and the mixture extracted exhaustively with dichloromethane. The combined organic layers were dried over sodium sulfate, filtered and the solvent evaporated giving 2,3-dihydro-2,2-dimethyl-4-(pyrrolidin-1-yl)pyridine (384 mg, 2.15 mmol). This reacted for 8 days at r.t. with benzyl bromide (810 mg, 4.74 mmol) in 20 ml of chloroform in the presence of potassium carbonate (2 g). Charcoal was added to the mixture and then it was heated till it boils. It was filtered and the solvent evaporated. The residue was dissolved with acetone and ethyl acetate was added till the mixture gets turbid. Upon stirring at room temperature a precipitate was formed which was sucked off and recrystallized from acetone as white powder. Yield: 436 mg, (46%). M.p.: 246°C; ¹H NMR (DMSO-d₆, *δ*, 400 MHz): 1.12 (s, 3H, CH₃), 1.16 (quin, *J* = 6.3 Hz, 1H, CH₂), 1.42 (s, 3H, CH₃), 1.53 - 1.65 (m, 2H, CH₂), 1.80 (quin, *J* = 6.6 Hz, 1H, CH₂), 2.14 (quin, *J* = 6.3 Hz, 1H, NCH₂), 2.49 (dd, *J* = 13.3, 9.4 Hz, 1H, ArC*H*₂CH), 2.98 (dd, *J* = 12.9, 4.9 Hz, 1H, ArC*H*₂CH), 3.13 (dd, *J* = 9.6, 4.9 Hz, 1H, 3H), 3.31 (quin, *J* = 6.6 Hz, 1H, NCH₂), 3.46 - 3.51 (m, 2H, NCH₂), 4.73 (d, *J* = 15.2 Hz, 1H, ArCH₂N), 4.86 (d, *J* = 15.2 Hz, 1H, ArCH₂N), 5.26 (d, *J* = 6.6 Hz, 1H, 5-H), 7.14 - 7.44 (m, 10H, ArH), 7.91 (d, *J* = 6.6 Hz, 1H, 6-H) ppm.

### (3RS)-(±)-1,3-Dibenzyl-2,2-dimethylpiperidin-4-one:

(3RS)-(±)-N-(1,3-Dibenzyl-2,2-dimethyl-1,2,3,4-tetrahydropyridin-4-ylidene)pyrrolidinium bromide (398 mg, 0.91 mmol) in 30 ml of dry diethyl ether, reacted with 144 mg (3.8 mmol) of LiAlH₄ giving after purification (CC, CH₂Cl₂:CH₃OH=20:1) 149 mg (53%) of an orange oil which solidifies. M.p. 74°C; ¹H NMR (CDCl₃, *δ*, 400 MHz): 1.12 (s, 3H, CH₃), 1.34 (s, 3H, CH₃), 2.27 - 2.38 (m, 2H, 5-H), 2.70 - 2.85 (m, 4H, 3-H, 6-H, ArCH₂), 3.27 (dd, *J* = 13.9, 9.9 Hz, 1H, ArCH₂), 3.65 (d, *J* = 13.9 Hz, 1H, ArCH₂N), 3.71 (d, *J* = 13.9 Hz, 1H, ArCH₂N), 7.14 - 7.42 (m, 10H, ArH) ppm.

### Example 3: (2RS,3RS)-(±)-3-Benzyl-2-isopropyl-1-(2-phenethyl)piperidin-4-one and (2RS,3SR)-(±)-3-benzyl-2-isopropyl-1-(2-phenethyl)piperidin-4-one

### (2RS,3RS)-(±)-N-(3-Benzyl-2-isopropyl-1-(2-phenylethyl)-1,2,3,4-tetrahydropyridin-4-ylidene)-N,N-dimethylammoniumiodide and (2RS,3SR)-(±)-N-(3-benzyl-2-isopropyl-1-(2-phenylethyl)-1,2,3,4-tetrahydropyridin-4-ylidene)-N,N-dimethylammoniumiodide:

(2RS)-(±)-N-(2-Isopropyl-1-(2-phenylethyl)-1,2,3,4-tetrahydropyridin-4-yliden)-N,N-dimethyl-ammoniumiodide (2.0 g, 5.69 mmol) was dissolved in 100 ml of chloroform, benzyl bromide (1.161 g, 6.84 mmol) and potassium carbonate (7.4 g) were added and the mixture refluxed at 80°C on an oil bath overnight. Then 100 ml of chloroform were added and the mixture was treated with charcoal and filtered. The solvent was evaporated in vacuo and the residue dissolved in acetone and ethyl acetate was added till the solution gets turbid. Upon stirring on an ice bath, a beige powder formed. Yield: 1.421 g (57%). M.p.: 165°C, ¹H NMR (DMSO-d₆, *δ*, 400 MHz): 0.75 (d, *J* = 6.6 Hz, 3H, CH(C*H*₃)₂), 0.84 (d, *J* = 7.0 Hz, 3H, CH(C*H*₃)₂), 2.04 (sex, *J* = 6.6 Hz, 1H, C*H*(CH₃), 2.70 (d, *J* = 7.7 Hz, 2H, ArCH₂), 2.88 (s, 3H, NCH₃), 2.91 - 3.08 (m, 2H, (ArC*H*₂CH₂), 3.06 (s, 3H, NCH₃), 3.29 (dd, *J* = 7.7, 7.3 Hz, 1H, 3-H), 3.41 (d, *J* = 5.9 Hz, 1H, 2-H), 3.62 (ddd, *J* = 13.9, 9.2, 5.5 Hz, 1H, NCH₂), 3.90 (ddd, *J* = 13.9, 9,5, 6.3 Hz, 1H, NCH₂), 5.21 (d, *J* = 7.0 Hz, 1H, 5-H), 7.21 - 7.35 (m, 10H, ArH), 7.90 (d, *J* = 7.0 Hz, 1H, 6-H) ppm.

### (2RS,3RS)-(±)-3-Benzyl-2-isopropyl-1-(2-phenethyl)piperidin-4-one and (2RS,3SR)-(±)-3-benzyl-2-isopropyl-1-(2-phenethyl)piperidin-4-one:

(2RS,3RS)-(±)-N-(3-Benzyl-2-isopropyl-1-(2-phenylethyl)-1,2,3,4-tetrahydropyridin-4-ylidene)-N,N-dimethylammoniumiodide and (2RS,3SR)-(±)-N-(3-Benzyl-2-isopropyl-1-(2-phenylethyl)-1,2,3,4-tetrahydropyridin-4-ylidene)-N,N-dimethylammoniumiodide (1.0 g, 2.27 mmol) in 70 ml of dry diethyl ether reacted with 360 mg (9.49 mmol) of LiAlH₄ giving after purification (CC, CH₂Cl₂:CH₃OH=13:1) 552 mg (72%) of a slightly orange oil. ¹H NMR (CDCl₃, *δ*, 400 MHz): 0.73 (d, *J* = 6.6 Hz, 3H, CH(C*H*₃)₂), 0.74 (d, *J* = 7.0 Hz, 3H, CH(C*H*₃)₂), 1.81 (sex, *J* = 6.8 Hz, 1H, C*H*(CH₃)₂), 2.23 (dt, *J* = 15.4, 2.6 Hz, 1H, 5-H), 2.42 (d, *J* = 7.3 Hz, 1H, 2-H), 2.63 - 2.71 (m, 2H, 3-H, 5-H), 2.76 - 3.14 (m, 8H, 6-H, ArCH₂, ArC*H₂*CH₂, NCH₂), 7.01 (d, *J* = 7.3 Hz, 2H, ArH), 7.14 - 7.33 (m, 8H, ArH) ppm.

### Example 4: (3RS)-(±)-3-Benzyl-1,2,2-trimethylpiperidin-4-one

### N-(1,2,2-Trimethyl-1,2,3,4-tetrahydropyridin-4-ylidene)pyrrolidinium iodide:

2,3-Dihydro-2,2-dimethyl-4-(pyrrolidin-1-yl)pyridine (960 mg g, 5.4 mmol) was dissolved in 27 ml of chloroform and methyl iodide (920 mg, 6.5 mmol) was added. The mixture was stirred overnight at room temperature. Then, the volume was reduced by evaporation of a part of the solvent and ethyl acetate was added dropwise. The product crystallized overnight as beige precipitate. Yield: 1.45 g (84%). M.p.: 138°C, ¹H NMR (DMSO-d₆, *δ*, 400 MHz): 1.28 (s, 6H, 2CH₃), 1.92 - 1.98 (m, 4H, (CH₂)₂), 2.87 (s, 2H, 3-H), 3.14 (s, 3H, NCH₃), 3.45 - 3.48 (m, 2H, NCH₂), 3.62 - 3.66 (m, 2H, NCH₂), 5.10 (d, *J* = 7.0 Hz, 1H, 5-H), 7.59 (d, *J* = 6.6 Hz, 1H, 6-H) ppm.

### (3RS)-(±)-N-(3-Benzyl-1,2,2-trimethyl-1,2,3,4-tetrahydropyridin-4-ylidene)pyrrolidiniumiodide:

N-(1,2,2-Trimethyl-1,2,3,4-tetrahydropyridin-4-ylidene)pyrrolidinium iodide (2.0 g, 6.24 mmol) was dissolved in 110 ml of chloroform, benzyl bromide (1.28 g, 7.488 mmol) and potassium carbonate (8 g) were added and the mixture refluxed at 80°C on an oil bath overnight. Then 110 ml of chloroform were added and the mixture was treated with charcoal and filtered. The solvent was evaporated in vacuo and the residue dissolved in acetone and ethyl acetate was added till the solution gets turbid. Upon stirring on an ice bath, a grey precipitate was formed. Yield 1.235 g (59%). M.p.: 228°C; ¹H NMR (DMSO-d₆, *δ*, 400 MHz): 1.10 - 1.17 (m, 4H, CH₂, CH₃), 1.49 - 1.63 (m, 5H, CH₂, CH₃), 1.77 (quin, *J* = 6.6 Hz, 1H, CH₂), 2.12 (quin, *J* = 7.0 Hz, 1H, NCH₂), 2.44 - 2.50 (m, 1H, ArCH₂), 3.07 - 3.24 (m, 6H, 3-H, ArCH₂, NCH₂, NCH₃), 3.42 (sept, *J* = 6.6 Hz, 2H, NCH₂), 5.10 (d, *J* = 7.0 Hz, 1H, 5-H), 7.17 - 7.31 (m, 5H, ArH), 7.65 (d, *J* = 6.6 Hz, 1H, 6-H) ppm.

### (3RS)-(±)-3-Benzyl-1,2,2-trimethylpiperidin-4-one:

(3RS)-(±)-N-(3-Benzyl-1,2,2-trimethyl-1,2,3,4-tetrahydropyridin-4-ylidene)pyrrolidiniumiodide (1.0 g, 2.75 mmol) in 90 ml of dry diethyl ether, reacted with 430 mg (11.93 mmol) of LiAlH₄ giving after purification (CC, CH₂Cl₂:CH₃OH=10:2) 236 mg (37%) of a brown oil of which solidifies to a crystalline solid giving needles. M.p.: 43°C; ¹H NMR (CDCl₃, *δ*, 400 MHz): 0.98 (s, 3H, CH₃), 1.24 (s, 3H, CH₃), 2.39 (s, 3H, NCH₃), 2.41 - 2.52 (m, 2H, 5-H), 2.69 (td, *J* = 11.1, 2.8 Hz, 2H, 3-H, ArCH₂), 2.91 (dd, *J* = 7.3, 5.5 Hz, 2H, 6-H), 3.18 (dd, *J* = 14.3, 10.3 Hz, 1H, ArCH₂), 7.12 - 7.26 (m, 5H, ArH) ppm.

### Example 5: 1-(2-Hydroxyethyl)-2,2-dimethylpiperidin-4-one

### N-(1-(Ethoxycarbonyl)methyl-2,2-dimethyl-1,2,3,4-tetrahydropyridin-4-ylidene)pyrrolidiniumbromide:

2,3-Dihydro-2,2-dimethyl-4-(pyrrolidin-1-yl)-pyridine (921 mg, 5.17 mmol) was dissolved in 25 ml of chloroform and ethyl-2-bromoacetate (1.467 g, 8.78 mmol) was added. The mixture was stirred for 5 days at room temperature. Then it was treated with charcoal, filtered and the solvent evaporated to dryness. The residue was dissolved in chloroform. Ethyl acetate was added dropwise until first turbidity appears. It was cooled with an ice bath and the product precipitated as off white lamellae. Yield: 1.123 g (63%). M.p.: 123°C; ¹H NMR (DMSO-d₆, *δ*, 400 MHz): 1.22 (t, *J* = 7.1 Hz, 3H, OCH₂C*H*₃), 1.26 (s, 6H, 2CH₃), 1.93 - 1.99 (m, 4H, (CH₂)₂), 2.92 (s, 2H, 3-H), 3.54 (t, *J* = 6.4 Hz, 2H, NCH₂), 3.69 (t, *J* = 6.4 Hz, 2H, NCH₂), 4.17 (q, *J* = 7.1 Hz, 2H, OC*H*₂CH₃), 4.43 (s, 2H, C*H*₂COOCH₂CH₃), 5.27 (d, *J* = 7.0 Hz, 1H, 5-H), 7.55 (d, *J* = 7.0 Hz, 1H, 6-H) ppm.

### 1-(2-Hydroxyethyl)-2,2-dimethylpiperidin-4-one:

N-(1-(Ethoxycarbonyl)methyl-2,2-dimethyl-1,2,3,4-tetrahydropyridin-4-ylidene)pyrrolidiniumbromide (1.054 g, 3.05 mmol) in 95 ml of dry diethyl ether reacted with 474 mg (12.04 mmol) of LiAlH₄ giving after purification (CC, CH₂Cl₂:CH₃OH=13:1) 269 mg (52%) of an orange oil. ¹H NMR (CDCl₃, *δ*, 400 MHz): 1.12 (s, 6H, 2CH₃), 2.35 (s, 2H, 3-H), 2.44 (t, *J* = 6.2 Hz, 2H, 5-H), 2.68 (t, *J* = 5.5 Hz, 2H, NCH₂), 2.95 (t, *J* = 6.2 Hz, 2H, 6-H), 3.00 (s, 1H, OH), 3.63 (t, *J* = 5.5, 2H, OCH₂) ppm.

### Example 6: 1-Benzyl-2,2-dimethyl-piperidin-4-one

### 5,6-Dihydro-6,6-dimethyl-4-(piperidin-1-yl)pyridine-2(1H)-thione:

A mixture of piperidinium thiocyanate (32.5 g, 0.23 mol) and mesityloxide (45 g, 0.45 mol) in 175 ml bromobenzene was refluxed over a water separator for 4h. Some solvent was removed by evaporation in vacuo. The product crystallized overnight. It was recrystallized from 2-propanol as bright yellow needles. Yield: 27.4 g (54%). M.p.: 217°C.

### N-(2,2-Dimethyl-6-methylsulfanyl-1,2,3,4-tetrahydropyridin-4-ylidene)piperidiniumiodide:

5,6-Dihydro-6,6-dimethyl-4-(1-piperidinyl)-2(*1H*)-pyridinthione (15g, 0.067 mol) was suspended in 20 ml of chloroform. Under stirring and cooling on an ice bath, methyl iodide (11.4 g, 0.08 mol) in 20 ml of chloroform was added dropwise. The mixture was stirred at room temperature overnight and the product precipitated with ethyl acetate as yellow solid. Yield: 23.7 g (97%). M.p.: 188°C

### N-(2,2-Dimethyl-1,2,3,4-tetrahydropyridin-4-ylidene)piperidiniumiodide:

N-(2,2-Dimethyl-6-methylsulfanyl-1,2,3,4-tetrahydropyridin-4-ylidene)piperidiniumiodide (20 g, 0.546 mol) was treated with raney nickel from 55 g nickel/aluminium alloy and 110 g of caustic soda giving 10.9 g (62%) as yellow needles. M.p. 133°C, ¹H NMR (DMSO-d₆, *δ*, 400 MHz): 1.26 (s, 6H, 2CH₃), 1.60 (s, br, 6H, 3CH₂), 2.85 (s, 2H, 3-H), 3.66 (s, br, 4H N(CH₂)₂), 5.38 (d, *J* = 6.2 Hz, 1H, 5-H), 7.60 (d, *J* = 5.5 Hz, 1H, 6-H), 9.32 (br, s, 1H, 1-H) ppm.

### N-(1-Benzyl-2,2-dimethyl-1,2,3,4-tetrahydropyridin-4-ylidene)piperidiniumbromide:

N-(2,2-Dimethyl-1,2,3,4-tetrahydropyridin-4-ylidene)piperidiniumiodide (930 mg, 2.9 mmol) was stirred for half an hour with 2N NaOH and the mixture extracted exhaustively with dichloromethane. The combined organic layers were dried over sodium sulfate, filtered and the solvent evaporated giving 2,3-dihydro-2,2-dimethyl-pyridin-4-yl)piperidin (563 mg, 2.9 mmol). This was reacted with benzyl bromide (820 mg, 4.8 mmol) in 3 ml of chloroform to 856 g (80%) as yellow crystals. M.p. 207°C, ¹H NMR (DMSO-d₆, *δ*, 400 MHz): 1.21 (s, 6H, 2CH₃), 1.62 (br, s, 6H, 3CH₂), 2.96 (s, 2H, 3-H), 3.69 (s, br, 4H, N(CH₂)₂), 4.77 (s, 2H, ArCH₂), 5.54 (d, *J* = 6.6 Hz, 1H, 5-H), 7.32 - 7.39 (m, 5H, ArH), 7.81 (d, *J* = 6.6 Hz, 1H, 6-H) ppm.

### 1-Benzyl-2,2-dimethyl-piperidin-4-one:

N-(1-Benzyl-2,2-dimethyl-1,2,3,4-tetrahydropyridin-4-ylidene)piperidiniumbromide (1.142 g 3.14 mmol) reacted in 100 ml of dry diethyl ether, with 505 mg (13.31 mmol) of LiAlH₄ giving after purification (CC, CH₂Cl₂) 501 mg (73%) of an orange oil which solidifies. M.p. 57°C; ¹H NMR (CDCl₃, *δ*, 400 MHz): 1.19 (s, 6H, 2CH₃), 2.33 (t, *J* = 6.0 Hz, 2H, 5-H), 2.39 (s, 2H, 3-H), 2.76 (t, *J* = 6.2 Hz, 2H, 6-H), 3.63 (s, 2H, ArCH₂), 7.24 - 7.40 (m, 5H, ArH) ppm.

### Example 7: 1-(4-Chlorobenzyl)-2,2-dimethyl-piperidin-4-one

### N-(1-(4-chlorobenzyl)-2,2-dimethyl-1,2,3,4-tetrahydropyridin-4-ylidene)pyrrolidinium bromide:

2,3-Dihydro-2,2-dimethyl-4-(pyrrolidin-1-yl)pyridine (785 mg, 4.4 mmol) was dissolved in 10 ml of chloroform and reacted with 4-chlorobenzylbromide (1.52 g, 7.4 mmol) by stirring overnight at room temperature. The solution was evaporated to dryness, dissolved in chloroform and cooled and ethyl acetate added till the solution gets turbid. In that way, 1.43 g (85%) of the product was yielded. ¹H NMR (DMSO-d₆, *δ*, 400 MHz): 1.21 (s, 6H, 2CH₃), 1.96 (br, s, 4H, (CH₂)₂), 2.90 (s, 2H, 3-H), 3.53 (br, s, 2H, NCH₂), 3.65 (br, s, 2H, NCH₂), 4.72 (s, 2H, ArCH₂), 5.25 (d, *J* = 7.2 Hz, 1H, 5-H), 7.34 - 7.46 (m, 4H, ArH), 7.74 (d, *J* = 7.2 Hz, 1H, 6-H) ppm.

### 1-(4-chlorobenzyl)-2,2-dimethyl-piperidin-4-one:

N-(1-(4-Chlorobenzyl)-2,2-dimethyl-1,2,3,4-tetrahydropyridin-4-ylidene)pyrrolidinium bromide (989 mg, 2.59 mmol) reacted in 80 ml of dry diethyl ether, with 400 mg (10.54 mmol) of LiAlH₄ and gave after purification (CC, CH₂Cl₂) 397 mg (61%) of an oil which solidifies at room temperature to a white solid. M.p.: 75°C; ¹H NMR (CDCl₃, *δ*, 400 MHz): 1.18 (s, 6H, 2CH₃), 2.32 (t, *J* = 6.4 Hz, 2H, 5-H), 2.38 (s, 2H, 3-H), 2.73 (t, *J* = 6.2 Hz, 2H, 6-H), 3.59 (s, 2H, ArCH₂), 7.29 (d, *J* = 8.4 Hz, 2H, ArH), 7.33 (d, *J* = 8.4 Hz, 2H, ArH) ppm.

### Example 8: (4RS)-N-(2,2-Dimethyl-1-(2-phenethyl)piperidin-4-yl)-N-phenylpropionamide via 2,2-Dimethyl-1-(2-phenylethyl)piperidin-4-one

### N-(2,2-Dimethyl-1-(2-phenylethyl)-1,2,3,4-tetrahydropyridin-4-ylidene)pyrrolidiniumiodide:

2,3-Dihydro-2,2-dimethyl-4-(pyrrolidin-1-yl)pyridine (5.756 g, 32.3 mmol) was dissolved in 40 ml of chloroform and 2-phenylethylbromide (15.7 g (84.84 mmol) dissolved in 50 ml of chloroform was added. Potassium carbonate (9g) was added and the mixture was stirred for 14 days at room temperature. After that, chloroform was added and the mixture was filtered and the solvent was evaporated in vacuo. The residue was dissolved in chloroform and ethyl acetate was added till the first turbidity remains. Upon cooling on a water bath stirring and adding more ethyl acetate, the product precipitated. Yield: 7.45 g (63%) as a beige powder. M.p. 204°C, ¹H NMR (DMSO-d₆, *δ*, 400 MHz): 1.32 (s, 6H, 2CH₃), 1.91 - 1.96 (m, 4H, (CH₂)₂), 2.89 - 2.93 (m, 4H, 3-H, ArCH₂), 3.47 (t, *J* = 5.9 Hz, 2H, NCH₂), 3.65 - 3.69 (m, 4H, NCH₂, ArCH₂C*H₂*N), 5.10 (d, *J* = 7.0 Hz, 1H, 5-H), 7.21 - 7.31 (m, 5H, ArH), 7.53 (d, *J* = 7.0 Hz, 1H, 6-H) ppm.

### 2,2-Dimethyl-1-(2-phenylethyl)piperidine-4-one:

N-(2,2-Dimethyl-1-(2-phenylethyl)-1,2,3,4-tetrahydropyridin-4-ylidene)pyrrolidiniumiodide (1.584 g, 4.36 mmol) reacted in 140 ml of dry diethyl ether, with 692 mg (18.23 mmol) of LiAlH₄ and gave after purification (CC, CH₂Cl₂:CH₃OH=20:1) 921 mg (91%) of a yellow oil. ¹H NMR (CDCl₃, *δ*, 400 MHz): 1.04 (s, 6H, 2CH₃), 2.28 (s, 2H, 3-H), 2.42 (t, *J* = 6.2 Hz, 2H, 5-H), 2.67 - 2.70 (m, 2H, NCH₂), 2.77 - 2.81 (m, 2H, ArCH₂), 2.96 (t, *J* = 6.2 Hz, 2H, 6-H), 7.19 - 7.31 (m, 5H, ArH) ppm.

### (4R,S)-2,2-Dimethyl-1-(2-phenylethyl)-N-phenylpiperidin-4-amine:

2,2-Dimethyl-1-(2-phenylethyl)piperidine-4-one (607 mg, 2.62 mmol) was dissolved in 7.5 ml of dry methanol. Aniline (1.207 g, 13 mmol) was added as well as sodium cyanoborohydride (333 mg, 5.3 mmol) dissolved in 3.7 ml of dry methanol. The pH-value was brought to 5 by dropwise addition of a mixture of glacial acetic acid/methanol = 1:3 and stirred for 20 min. After that, the pH-value was checked again and again brought to 5 by addition of the above mentioned mixture. This was repeated till the pH-value remained at 5. The mixture was stirred overnight. Then the same volume water was added and concentrated HCl was added dropwise to bring the mixture to pH = 1. Gas bubbles are formed and the mixture was stirred for 20 min at room temperature. Then the double volume brine was added and the mixture extracted 4 times with dichloromethane. The organic layers were shaken 3 times with 2N NaOH and once with brine, dried over sodium sulfate and filtered. The solvents were evaporated and the residue was purified by column chromatography over basic aluminum oxide using dichloromethane as eluent. The solvent was evaporated giving 136 mg (17%) of the product as brownish oil. ¹H NMR (CDCl₃, *δ*, 400 MHz): 0.93 (s, 3H, CH₃), 1.02 (s, 3H, 2CH₃), 1.21 (br, t, *J* = 12.3 Hz, 1H, 3-Hₐₓ), 1.32 (ddd, *J* = 12.5, 12.1, 4.0Hz, 1H, 5-Hₐₓ), 1.77 (ddd, *J* = 12.8, 3.0, 2.9 Hz, 1H, 3-H_{equ}), 2.02 (ddd, *J* = 12.8, 2.9, 2.6 Hz, 1H, 5-H_{equ}), 2.10 - 2.17 (m, 1H, NCH₂), 2.40 (dd, *J* = 12.6, 2.6 Hz, 1H, 6-Hₐₓ), 2.56 - 2.64 (m, 1H, ArCH₂) , 2.73 (ddd, *J* = 12.8, 10.5, 4.9 Hz, 1H, ArCH₂), 2.84 - 2.92 (m, 2H, 6-H_{equ}, NCH₂), 3.30 (br, s, 1H, NH), 3.40 (br, t, *J* = 11.4 Hz, 1H, 4-H), 6.51 (d, *J* = 8.4 Hz, 2H, ArH), 6.60 (t, *J* = 7.3 Hz, 1H, ArH), 7.05 - 7.23 (m, 7H, ArH) ppm.

### (4RS)-N-(2,2-Dimethyl-1-(2-phenylethyl)piperidin-4-yl)-N-phenylpropionamide:

(4RS)-2,2-Dimethyl-1-(2-phenethyl)-N-phenylpiperidin-4-amine (136 mg, 0.44 mmol) was dissolved in 2 ml of dry dichloromethane. To this solution, diisopropylethylamine (114 mg, 0.88 mmol) was added. The mixture was set under argon and cooled on an ice bath and a solution of propionylchloride (81 mg, 0.88 mmol) in 2 ml of dry dichloromethane was added dropwise using a syringe. The mixture was stirred for 2 h. Then 20 ml of dichloromethane was added and the solution poured in a separatory funnel and shaken with the same volume of water. The organic layer was washed with brine and a saturated solution of NaHCO₃. Then it was dried over sodium sulfate, filtered, and the solvent removed in vacuo. The residue was subjected to column chromatography over silicagel using CH₂Cl₂:CH₃OH=20:1 as eluent. Yield: 87 mg (54 %) of a yellowish oil. Due to the partial double bond character of the amide bond, two isomers were detected.

Minor constituent (*Z*-form): ¹H NMR (CDCl₃, *δ*, 400 MHz): 0.97 - 1.08 (m, 6H, 2CH₃), 1.12 (t, *J* = 7.7 Hz, 3H, C*H*₃CH₂), 1.50 - 1.55 (m, 1H, 3-Hₐₓ), 1.56 - 1.63 (m, 1H, 5-Hₐₓ), 1.69 - 1.73 (m, 1H, 3-H_{eq}), 1.96 - 1.99 (m, 1H, 5-H_{eq}), 2.26 - 2.30 (m, 1H, NCH₂), 2.29 (q, *J* = 7.7 Hz, 2H, CH₃CH₂), 2.41 - 2.60 (m, 2H, 6-Hₐₓ, ArCH₂), 2.64 - 2.81 (m, 2H, ArCH₂, NCH₂), 2.82 - 2.97 (m, 1H, 6-H_{eq}), 4.88 - 4.98 (m, 1H, 4-H), 7.06 - 7.41 (m, 10H, ArH) ppm.

Main component (E-form): ¹H NMR (CDCl₃, *δ*, 400 MHz): 0.97 - 1.08 (m, 9H, CH₃CH₂, 2CH₃), 1.20 - 1.36 (m, 2H, 3-Hₐₓ, 5-Hₐₓ), 1.59 (ddd, *J* = 12.1, 2.9, 2.9 Hz, 1H, H-3_{eq}), 1.77 - 1.81 (m, 1H, 5-H_{eq}), 1.91 (q, *J* = 7.3 Hz, 2H, CH₃C*H*₂), 2.13 (ddd, *J* = 12.0, 11.1, 4.8 Hz, 1H, NCH₂), 2.41 - 2.60 (m, 2H, 6-Hₐₓ, ArCH₂), 2.64 - 2.81 (m, 1H, ArCH₂), 2.82 - 2.97 (m, 2H, 6-H_{eq}, NCH₂), 4.89 - 4.98 (m, 1H, 4-H), 7.06 - 7.41 (m, 10H, ArH) ppm.

### Example 9: 1-(2-(1H-Indol-3-yl)ethyl)-2,2-dimethylpiperidine-4-one

### 5,6-Dihydro-6,6-dimethyl-4-(pyrrolidin-1-yl)-2(1H)-thiopyranthione:

To ice cooled pyrrolidine (71.1g, 1 mol), CS₂ (38g, 0.5 mol) was added dropwise, followed by the dropwise addition of mesityloxide (49g, 0.5 mol). The mixture was refluxed on a water separator for 4 h and the product precipitated upon cooling. It was recrystallized from ethanol. Yield: 82g (72%). M.p.: 233°C.

### N-(3,4-Dihydro-2,2-dimethyl-6-methysulfanyl-2H-thiopyran-4-yliden)pyrrolidiniumiodide:

5,6-Dihydro-6,6-dimethyl-4-(pyrrolidin-1-yl)-2(*1H*)-thiopyranthione (47.5g, 0.2 mol) was dissolved in 75 ml of chloroform. A solution of methyl iodide (58.5g, 0.4 mol) in 75 ml chloroform was added dropwise and the mixture stirred overnight at room temperature. The solvent was removed by evaporation in vacuo and the residue triturated with ethyl acetate.

Yield: 71.3 (96%) of a yellow powder. M.p.: 198°C.

### N-(5,6-Dihydro-6,6-dimethyl-4-(pyrrolidin-1-yl)-2H-thiopyran-2-ylidene)-2-(1H-indol-3-yl)ethanamine:

N-(3,4-Dihydro-2,2-dimethyl-6-methylsulfanyl-2H-thiopyran-4-ylidene)pyrrolidinium iodide (20g, 54 mmol) and tryptamine (8.7g, 54 mmol) were dissolved in the minimum amount of ethanol. The solution was stirred at room temperature for 5 days while compressed air was bubbled through the solution. The remaining residue was dissolved in ethanol cooled on an ice bath and treated with ethyl acetate. The product appeared as red oil, the solvent was decanted and residual solvent was removed in vacuo giving as red foam (15.1g, 58%). This foam was dissolved with ethanol and 2N NaOH was added till the solution gets turbid. Then it was extracted exhaustively with ether. The combined organic phases were dried over sodium sulfate, filtered and the solvent removed in vacuo giving the product as an orange foam.

### 5,6-Dihydro-1-(2-(1H-indol-3-yl)ethyl)-6,6-dimethyl-4-(pyrrolidin-1-yl)pyridine-2(1H)-thione:

N-(5,6-Dihydro-6,6-dimethyl-4-(pyrrolidin-1-yl)-2H-thiopyran-2-ylidene)-2-(1*H*-indol-3-yl)ethanamine (15g, 42 mmol) was dissolved in 200 ml dimethylformamide and refluxed for 16 h. Then the solvent was removed in vacuo and the residue recrystallized from ethanol giving the product (5g, 33%) as beige crystals. M.p. 261°C; ¹H NMR (DMSO-d₆, *δ*, 400 MHz): 1.37 (s, 6H, 2CH₃) , 1.85 (br, s, 4H, 2CH₂) , 2.56 (s, 2H, 5-H) , 3.06 (t, *J* = 8.1 Hz, 2H, 2'-H), 3.10 - 3.40 (m, 4H, 2NCH₂) , 4.09 (br, s, 2H, 1'-H), 5.39 (s, 1H, 3-H), 6.97 (dd, *J* = 7.3, 7.3 Hz, 1H, ArH), 7.05 (dd, *J* = 7.7, 7.3 Hz, 1H, ArH), 7.16 (s, 1H, ArH), 7.33 (d, *J* = 8.1 Hz, 1H, ArH), 7.81 (d, *J* = 7.7 Hz, 1H, ArH), 10.80 (s, 1H, NH) ppm.

### N-(1-(2-(1H-Indol-3-yl)ethyl)-2,2-dimethyl-6-methylsulfanyl-1,2,3,4-tetrahydropyridin-4-ylidene)pyrrolidiniumiodide:

5,6-Dihydro-1-(2-(1*H*-indol-3-yl)ethyl)-6,6-dimethyl-4-(pyrrolidin-1-yl)pyridine-2(1*H*)-thione (5g, 14.1 mmol) was dissolved in 10 ml of chloroform and methyl iodide (2.4g, 17 mmol) dissolved in 10 ml of chloroform was added dropwise. The solution was stirred overnight at room temperature. The solvent was evaporated and the residue recrystallized from chloroform/ethylacetate giving the product (5.8 g, 83%) as white crystals. M.p. 211°C; ¹H NMR (DMSO-d₆, *δ*, 400 MHz): 1.44 (s, 6H, 2CH₃), 1.94 - 2.01 (m, 4H, 2CH₂), 2.67 (s, 3H, SCH₃), 2.91 (s, 2H, 3-H), 3.07 (t, *J* = 8.2 Hz, 2H, 2'-H), 3.56 (br, t, *J* = 6.2 Hz, 2H, NCH₂), 3.62 (br, t, *J* = 6.4 Hz, 2H, NCH₂), 3.79 (t, *J* = 8.4 Hz, 2H, 1'-H), 5.18 (s, 1H, 5-H), 7.01 (dd, *J* = 7.7, 7.3 Hz, 1H, ArH), 7.09 (dd, *J* = 7.7, 7.3 Hz, 1H, ArH), 7.27 (s, 1H, ArH), 7.36 (d, *J* = 8.1 Hz, 1H, ArH), 7.57 (d, *J* = 7.7 Hz, 1H, ArH), 10.97 (br, s, 1H, NH) ppm.

### N-(1-(2-(1H-Indol-3-yl)ethyl)-2,2-dimethyl-1,2, 3,4-tetrahydropyridin-4-ylidene)pyrrolidiniumiodide:

N-(1-(2-(1*H*-Indol-3-yl)ethyl)-2,2-dimethyl-6-methylsulfanyl-1,2,3,4-tetrahydropyridin-4-ylidene)pyrrolidiniumiodide (1.066 g, 2.15 mmol) reacted with raney nickel prepared from 12 g powdered nickel/aluminium alloy in 20 ml ethanol to a white foam which was recrystallized from ethanol. Yield: 500 mg (61%). M.p.: (ethanol): 206°C. ¹H NMR (DMSO-d₆, *δ*, 400 MHz): 1.35 (s, 6H, 2CH₃), 1.91 - 1.97 (m, 4H, 2CH₂), 2.87 (s, 2H, 3-H), 3.03 (t, *J* = 7.3 Hz, 2H, 2'-H), 3.46 (br, t, *J* = 5.5 Hz, 2H, NCH₂), 3.63 (br, t, *J* = 5.5 Hz, 2H, NCH₂), 3.70 (t, *J* = 7.3 Hz, 2H, 1'-H), 5.08 (d, *J* = 7.0 Hz, 1H, 5-H), 6.99 (dd, *J* = 7.7, 7.0 Hz, 1H, ArH), 7.08 (dd, *J* = 7.7, 7.3 Hz, 1H, ArH), 7.23 (s, 1H, ArH), 7.35 (d, *J* = 8.1 Hz, 1H, ArH), 7.42 (d, *J* = 7.0 Hz, 1H, 6-H), 7.59 (d, *J* = 7.7 Hz, 1H, ArH), 10.91 (br, s, 1H, NH) ppm.

### 1-(2-(1H-Indol-3-yl)ethyl)-2,2-dimethylpiperidine-4-one:

N-(1-(2-(1*H*-Indol-3-yl)ethyl)-2,2-dimethyl-1,2,3,4-tetrahydropyridin-4-ylidene)pyrrolidiniumiodide (378 mg, 0.84 mmol) reacted in 30 ml of dry diethyl ether with 135 mg (3.57 mmol) of LiAlH₄ giving after purification (CC, (CH₂Cl₂:CH₃OH) 70 mg (31%) of an brown oil. ¹H NMR (CDCl₃, *δ*, 400 MHz): 1.08 (s, 6H, 2CH₃), 2.33 (s, 2H, 3-H), 2.42 - 2.50 (m, 2H, 5-H), 2.78 (br, dd, *J* = 8.8, 7.0 Hz, 2H, 1'-H), 2.98 (br, t, *J =* 7.7 Hz, 2H, 2'-H), 3.04 (br, t, *J* = 6.0 Hz, 2H, 6-H), 7.38 (d, *J* = 1.5 Hz, 1H, ArH), 7.12 (dd, *J* = 7.7, 7.0 Hz, 1H, ArH), 7.20 (dd, *J* = 8.1, 7.0 Hz, 1H, ArH), 7.36 (d, *J* = 8.1 Hz, 1H, ArH), 7.61 (d, *J* = 7.7 Hz, 1H, Ar-H), 8.12 (br, s, 1H, NH) ppm.

## Claims

1. Process for preparing a piperidin-4-one comprising a step of reducing a tetrahydropyridin-4-ylidene ammonium salt of formula (I) 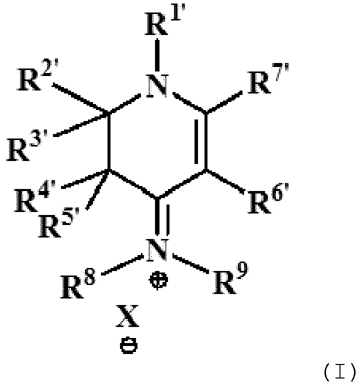 to a compound of formula (II) 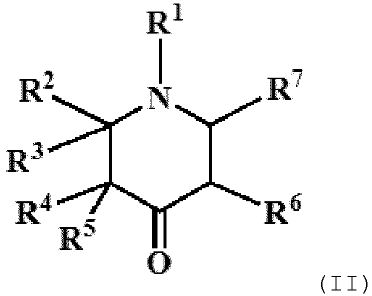 wherein R¹, R², R³, R⁴, R⁵, R⁶, and R⁷ are independently from each other substituents selected from the group consisting of
a) hydrogen,
b) C₁-C₁₀-alkyl,
c) C₆-C₁₄-aryl,
d) heteroaryl, wherein the heteroaryl is 5 to 14 membered aromatic ring system with at least one heteroatom selected from the group consisting of N, O, and S atoms,
e) C₅-C₁₄-aryl-C₁-C₄-alkyl,
f) heteroaryl-C₁-C₄-alkyl,
g) C₂-C₁₀-alkenyl containing one or more double bonds,
h) C₂-C₁₀-alkinyl containing one or more triple bonds,
i) C₅-C₁₄-aryl-C₂-C₄-alkenyl containing one or more double bonds,
j) C₅-C₁₄-aryl-C₂-C₄-alkinyl containing one or more triple bonds,
k) heteroaryl-C₂-C₄-alkenyl containing one or more double bonds, and
l) heteroaryl-C₂-C₄-alkinyl containing one or more triple bonds, wherein the heteroaryl is 5 to 14 membered aromatic ring system with at least one heteroatom selected from the group consisting of N, O, and S atoms,
and wherein optionally one or more of substituents b) to 1) are substituted
R⁸ and R⁹ are independently from each other substituents selected from the group consisting of a) to 1) as defined above, or
R⁸ and R⁹ form together with the nitrogen to which they are bound a 3 to 7 membered unsubstituted or substituted non-aromatic heterocyclic ring, wherein the non-aromatic heterocyclic ring optionally contains one or more additional heteroatoms selected from the group consisting of N, O, and S atoms,
wherein R^{1'}, R^{2'}, R^{3'}, R^{4'}, R^{5'}, R^{6'}, and R^{7'} independently of each other are identical to R¹, R², R³, R⁴, R⁵, R⁶, and R⁷, respectively, or R^{1'}, R^{2'}, R^{3'}, R^{4'}, R^{5'}, R^{6'}, and R^{7'} are precursors of R¹, R², R³, R⁴, R⁵, R⁶, and R⁷, respectively, which are reducible to R¹, R², R³, R⁴, R⁵, R⁶, and R⁷, respectively,
and X⁻ is an anion.

2. Process according to claim 1, wherein the reducing step is performed using a hydrogenation agent.

3. Process according to claim 2, wherein the hydrogenation agent is an aluminium hydride, preferably selected from the group consisting of sodium bis(2-methoxyethoxy)aluminium hydride and lithium aluminium hydride, more preferably lithium aluminium hydride.

4. Process according to claim 2 or 3, wherein molar ratio between the hydrogenating agent and the amount of the tetrahydropyridin-4-ylidene ammonium salt of formula (I) is in the range of from 2:1 to 6:1, preferably from 3:1 to 5:1, more preferably from 3.5:1 to 4.5:1, more preferably from 3.8:1 to 4.4:1, more preferably about 4:1.

5. Process according to any one of claims 1 to 4, wherein at least one of R², R^{2'}, R³, R^{3'}, R⁷, or R^{7'} is not hydrogen, preferably wherein either R² and R^{2'} or R³ and R^{3'} or all of R², R^{2'}, R³ and R^{3'}, are not hydrogen.

6. A piperidin-4-one compound of formula (II) 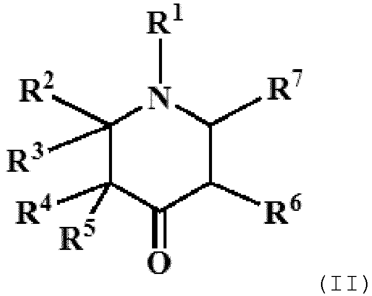 wherein R¹, R², R³, R⁴, R⁵, R⁶, and R⁷ are independently from each other substituents selected from the group consisting of
a) hydrogen,
b) C₁-C₁₀-alkyl,
c) C₆-C₁₄-aryl,
d) heteroaryl, wherein the heteroaryl is 5 to 14 membered aromatic ring system with at least one heteroatom selected from the group consisting of N, O, and S atoms,
e) C₅-C₁₄-aryl-C₁-C₄-alkyl,
f) heteroaryl-C₁-C₄-alkyl,
g) C₂-C₁₀-alkenyl containing one or more double bonds,
h) C₂-C₁₀-alkinyl containing one or more triple bonds,
i) C₅-C₁₄-aryl-C₂-C₄-alkenyl containing one or more double bonds,
j) C₅-C₁₄-aryl-C₂-C₄-alkinyl containing one or more triple bonds,
k) heteroaryl-C₂-C₄-alkenyl containing one or more double bonds,
l) heteroaryl-C₂-C₄-alkinyl containing one or more triple bonds, wherein the heteroaryl is 5 to 14 membered aromatic ring system with at least one heteroatom selected from the group consisting of N, O, and S atoms,
and wherein optionally one or more of substituents b) to 1) are substituted,
and wherein at least one of R², R³, or R⁷ is not hydrogen, preferably at least one of R² and R³ is not hydrogen.

7. Process according to any one of claims 1 to 5 or compound according to claim 6, wherein any substituent b) to 1) is substituted with one or more further substituent(s) selected from the group consisting of halogenide, cyano, hydroxyl, C₁-C₄-alkoxy, and C₁-C₄-alkyl.

8. Process according to any one of claims 1 to 5 or 7, or compound according to claim 6 or 7, wherein at least one of R⁵, R⁶, and R⁷ is hydrogen, preferably wherein all of R⁵, R⁶, and R⁷ are hydrogen.

9. Process according to claim 1 to 5 and 7 to 8 or compound according to claim 6 to 8, wherein R¹, R², R³, R⁴ are independently from each other are selected from the group consisting of hydrogen, C₁-C₆-alkyl, C₆-aryl, heteroaryl, C₆-aryl-C₁-C₄-alkyl and heteroaryl-C₁-C₄-alkyl and R⁵, R⁶, and R⁷ are hydrogen.

10. Process according to any one of claims 1 to 5 and 7 to 9 or compound according to any one of claims 6 to 9, wherein R² and R³ both are methyl and R¹ is selected from the group consisting of prop-2-yl, 1-methyl-2-phenethyl, 2-hydroxyethyl, 2-hydroxypropyl, 2-(1H-indol-3-yl)ethyl, pyridin-2-ylmethyl, 3-cyano-3-diphenylpropyl-, 4-oxo-4-(4-fluorphenyl)butyl, 4-di(4-fluorphenyl)butyl, 3-(4-fluorphenoxy)propyl, 2-(4-methoxyphenyl)ethyl, 3-(4-fluorphenoxy)-2-hydroxylpropyl, and 3-(2-oxo-1-benzimidazolinyl)propyl, preferably 2-(1H-indol-3-yl)ethyl.

11. Process according to any one of claims 1 to 5 and 7 to 9 or compound according to any one of claims 6 to 9, wherein R² and R³ both are methyl and R¹ is selected from the group consisting of hydrogen, methyl, isopropyl, n-butyl, benzyl, phenyl, 2-phenethyl, allyl, acetyl, methylsulfonyl, benzoyl and benzyl carboxylate, and at least one of R⁴, R⁵, R⁶, and R⁷ is not hydrogen, preferably, at least R⁴ is not hydrogen, preferably R⁴ is benzyl.

12. Process according to any one of claims 1 to 5 and 7 to 9 or compound according to any one of claims 6 to 9, wherein one of R² or R³ is isopropyl and the other one of R² or R³ is hydrogen and R¹ is selected from the group consisting of prop-2-yl, 2-phenethyl, 1-methyl-2-phenethyl, 2-hydroxyethyl, 2-hydroxypropyl, 2-(1H-indol-3-yl)ethyl, pyridin-2-ylmethyl, 3-cyano-3-diphenylpropyl-, 4-oxo-4-(4-fluorphenyl)butyl, 4-di(4-fluorphenyl)butyl, 3-(4-fluorphenoxy)propyl, 2-(4-methoxyphenyl)ethyl, 3-(4-fluorphenoxy)-2-hydroxylpropyl, and 3-(2-oxo-1-benzimidazolinyl)propyl, preferably 2-phenethyl, 2-(1H-indol-3-yl)ethyl.

13. Process according to any one of claims 1 to 5 and 7 to 9 or compound according to any one of claims 6 to 9, wherein one of R² or R³ is isopropyl and the other one of R² or R³ is hydrogen and R¹ selected from the group consisting of hydrogen, methyl, isobutyl, benzyl, phenyl, acetyl, methyl carboxylate, tert-butyl carboxylate, phenyl carboxylate and benzyl carboxylate, and at least one of R⁴, R⁵, R⁶, and R⁷ is not hydrogen, preferably, at least R⁴ is not hydrogen, preferably R⁴ is benzyl.

14. Process according claim 1 or compound according to claim 6, wherein the compound of formula (II) is selected from the group consisting of (2RS)-(±)-2-isopropyl-1-phenethylpiperidin-4-one, (2RS)-(±)-2-isopropyl-1-propylpiperidin-4-one, (2RS)-(±)-1-butyl-2-isopropylpiperidin-4-one, (2RS)-(±)-1-sec-butyl-2-isopropylpiperidin-4-one, (2RS)-(±)-1-(2-hydroxyethyl)-2-isopropylpiperidin-4-one, (2RS)-(±)-1-(2-hydroxypropyl)-2-isopropylpiperidin-4-one, (2RS)-(±)-2-isopropyl-1-vinylpiperidin-4-one, (2RS)-(±)-1-allyl-2-isopropylpiperidin-4-one, (2RS)-(±)-1-cyclohexyl-2-isopropylpiperidin-4-one, (2RS)-(±)-1-(cyclohexylmethyl)-2-isopropylpiperidin-4-one, 2,2-dimethyl-1-vinylpiperidin-4-one, 1-(2-hydroxyethyl)-2,2-dimethylpiperidin-4-one, and 1-(2-hydroxypropyl)-2,2-dimethylpiperidin-4-one, preferably selected from the group consisting of (2RS)-(±)-2-isopropyl-1-(2-phenylethyl)piperidine-4-one, (3RS)-(±)-1,3-dibenzyl-2,2-dimethylpiperidin-4-one, (2RS,3RS)-(±)-3-benzyl-2-isopropyl-1-(2-phenylethyl)piperidin-4-one, (2RS,3SR)-(±)-3-benzyl-2-isopropyl-1-(2-phenylethyl)piperidin-4-one, (3RS)-(±)-3-benzyl-1,2,2-trimethylpiperidin-4-one and 1-(2-hydroxyethyl)-2,2-dimethylpiperidin-4-one.

15. Method of derivatizing a piperidin-4-one comprising the step of subjecting a compound of formula (II) obtainable by a process of any one of claims 1 to 5 and 7 to 14 or a compound according to any one of claims 6 to 14 to a reaction selected from the group of
- reductive amination to obtain a compound according to formula (XIX) 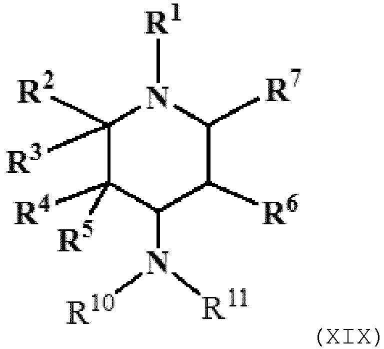
- reduction and etherification to obtain a compound according to formula (XX) 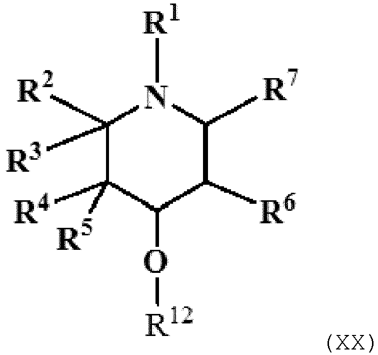
- reacting by Grignard reaction to obtain a compound according to formula (XXI) 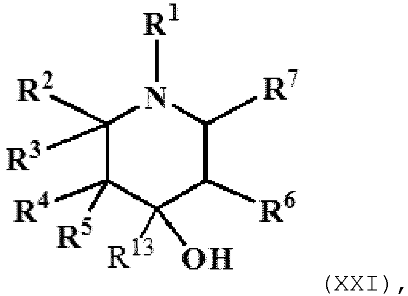
wherein R¹⁰, R¹¹, R¹² and R¹³ are independently of each other hydrogen or an organic moiety.
